# EUROPEAN PATENT APPLICATION

(11) **EP 4 582 429 A1**
(43) Date of publication of application: **09.07.2025**
(21) Application number: 23860520.8
(22) Date of filing: 31.08.2023
(51) Int. Cl.: C07D 487/16, G01N 23/20

(54) **POROUS NETWORK COMPLEX, METHOD FOR PRODUCING SAMPLE FOR CRYSTAL STRUCTURE ANALYSIS AND METHOD FOR DETERMINING MOLECULAR STRUCTURE**

(30) Priority: 02.09.2022 JP 2022140327
(71) Applicant: INSTITUTE OF SCIENCE TOKYO, Tokyo 152-8550 (JP)
(72) Inventor: KAWANO, Masaki, Tokyo 152-8550 (JP); WADA, Yuki, Tokyo 152-8550 (JP); USOV, Pavel, Tokyo 152-8550 (JP); TAGAMI, Yu, Tokyo 152-8550 (JP); ZHU, Yiying, Tokyo 152-8550 (JP)
(74) Representative: INNOV-GROUP
(86) International application number: PCT/JP2023/032004
(87) International publication number: WO 2024/048758

(57) **Abstract**

A porous coordination network is represented by the following formulas (I) to (VIII), in which M²⁺ is a divalent metal ion, M³⁺ is a trivalent metal ion, L1a and L1b are tridentate ligands having hexaazaphenalenyl, L2a and L2b are bidentate or tridentate ligands containing a carboxy group, L3⁻ is a tertiary ligand ion that is an anion of triazole or a triazole derivative, and Y is a cation.
(Chemical Formula 1)

(M²⁺)₄(L1a⁻)₂(L2a²⁻)₃ formula (I)

(M²⁺)₁₁(L1a⁻)₆(L2a²⁻)₈ formula (II)

[(M²⁺)₅(L1a⁻)₄(L2a²⁻)₄]·Y₂ₚ formula (III)

[(M²⁺)₂(L1a⁻)₁(L2a²⁻)₂]·Yₚ formula (IV)

(M²⁺)₂(L1a⁻)₁(L2b³⁻)₁ formula (V)

[(M²⁺)₃(L1a⁻)₂(L2b³⁻)₂]·Y₂ₚ formula (VI)

[(M²⁺)₈(L1b³⁻)₂(L2a²⁻)₃(L3⁻)₆]·Y₂ₚ formula (VII)

(M²⁺)₅(M³⁺)₁(L1a⁻)₃(L2a²⁻)₅ formula (VIII)

## Description

### Technical Field

The present disclosure relates to a porous coordination network. The present disclosure further relates to a method for preparing a sample for crystal structure analysis and a method for determining a molecular structure, using the porous coordination network.

### Background Art

A porous coordination network (PCN) including metal ions and organic ligands have an infinite structure that can maintain the structure after the solvent is removed, and the technology that utilizes the pores thereof has been proposed. For example, the PCN that undergoes reversible structural changes in response to the adsorption and desorption of guests in the pores (Non Patent Literature 1), the PCNs that combine a 2,4,6-tris(4-pyridyl)triazine ligand with ZnBr₂ (Non Patent Literatures 2 and 3), and the PCN that combines a tripyridylhexaazaphenalenate (K⁺TPHAP⁻) ligand with ZnI₂ has been reported (Non Patent Literature 4). In addition, the present inventors have recently reported a porous coordination network formed by coordinating a first ligand in which the nitrogen atom of a pyridinyl group functions as a ligand, and a second ligand to a metal (Non Patent Literature 5, Patent Literatures 1 and 2).

### Citation List

### Patent Literature

Patent Literature 1: Japanese Unexamined Patent Application Publication No. 2020-147521
Patent Literature 2: Japanese Unexamined Patent Application Publication No. 2022-11418

### Non Patent Literature

Non Patent Literature 1: Sujit K. Ghosh et al., Angew. Chem., 2013, 125, 1032
Non Patent Literature 2: Masaki Kawano et al., Angew. Chem. Int. Ed. 2008, 47, 1269-1271
Non Patent Literature 3: Makoto Fujita et al. NATURE CHEMISTRY, VOL 3, 2011
Non Patent Literature 4: Masaki Kawano, et al. CrystEngComm., 2014, 16, 6335
Non Patent Literature 5: Masaki Kawano, et al. Inorg. Chem., 2021, 60, 17858-17864

### Summary of Invention

### Technical Problem

A PCN has a very large surface area compared to zeolites and activated carbons, and the structure thereof can be customized to produce isotopically-ordered structures, and thus the PCN is attracting attention in the fields of catalyst support, adsorption, and separation and purification. In addition, if it were possible to provide a PCN capable of analyzing medium-sized molecules (molecules with a molecular weight of approximately 500 to 3,000), which have been considered difficult to analyze until now, it is expected that pharmaceutical development will be dramatically accelerated. However, as a result of extensive investigations, the inventors have found that in order to incorporate medium-sized molecules as guest molecules, it is not enough to consider the molecular size of the guest molecule and the size of the PCN, and it is also necessary to consider the compatibility between the shape of the guest molecule and the pore structure of the PCN. That is, it has been found that it is necessary not only to simply adjust the pore size but also to select a PCN appropriate for the chemical structure of the guest molecule. If various PCNs with different pore shapes could be provided, it would be possible to increase the number of medium-sized molecules with the structures capable of being analyzed using the PCN, which would make a significant contribution to the structural analysis of medium-sized molecules.

The case described above involves the use of a medium-sized molecule as the guest molecule; however, similar problems may occur regardless of the molecular weight.

The present disclosure has been made in view of the above background, and the object thereof is to provide a porous coordination network having a novel structure, a method for preparing a sample for crystal structure analysis, and a method for determining a molecular structure.

### Solution to Problem

As a result of extensive investigations, the present inventors have found that the problems of the present disclosure can be solved in the following aspect, and have thus completed the present disclosure.
[1] A porous coordination network which has a three-dimensional network structure and in which pores capable of encapsulating guest molecules are formed,
   the porous coordination network including a crystal structure having a unit structure represented by any one of the following formulas (I) to (VIII):

      (M²⁺)ₐ(L1a⁻)₂(L2a²⁻)₃ formula (I)

      (M²⁺)₁₁(L1a⁻)₆(L2a²⁻)₈ formula (II)

      [(M²⁺)₅(L1a⁻)₄(L2a²⁻)₄]·Y₂ₚ formula (III)

      [(M²⁺)₂(L1a)₁(L2a²⁻)₂]·Yₚ formula (IV)

      (M²⁺)₂(L1a⁻)₁(L2b³⁻)₁ formula (V)

      [(M²⁺)₃(L1a⁻)₂(L2b³⁻)₂]·Y₂ₚ formula (VI)

      [(M²⁺)₈(L1b³⁻)₂(L2a²⁻)₃(L3⁻)₆]·Y₂ₚ formula (VII)

      (M²⁺)₅(M³⁺)₁(L1a⁻)₃(L2a²⁻)₅ formula (VIII)
   where M²⁺ represents a divalent metal ion, M³⁺ represents a trivalent metal ion,
   L1a and L1b represent tridentate ligands having hexaazaphenalenyl,
   L1a⁻ represents a first ligand ion of the following formula (1), or a first ligand ion in which 1 to 12 hydrogen atoms of an aromatic ring of the formula (1) are each independently replaced by a group selected from an alkyl group having 1 to 4 carbon atoms, a halogen, a hydroxyl group, and an amino group, 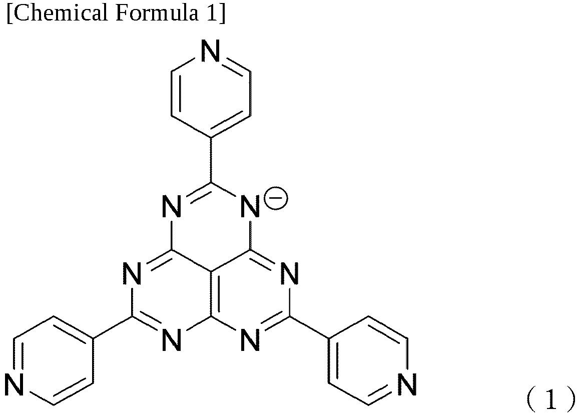
   L1b³⁻ represents a first ligand ion represented by the following formula (2), or a first ligand ion in which 1 to 12 hydrogen atoms of an aromatic ring of the formula (2) are each independently replaced by a group selected from an alkyl group having 1 to 4 carbon atoms, a halogen, a hydroxyl group, and an amino group, 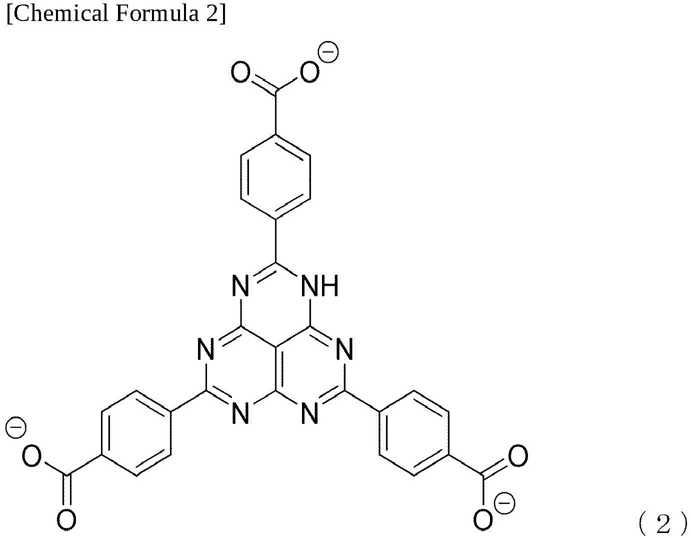
   L2a represents a bidentate ligand containing two or more carboxy groups,
   L2a²⁻ represents a second ligand ion in which two of the carboxy groups are coordinated to the metal ion as carboxylate anions,
   L2b represents a tridentate ligand containing three or more carboxy groups,
   L2b³⁻ represents a second ligand ion in which three of the carboxy groups are coordinated to the metal ion as carboxylate anions,
   L3⁻ represents a third ligand ion that is an anion of triazole or a triazole derivative,
   Y represents a cation, p represents 1/(charge of Y), and
   a solvent is optionally further coordinated to the unit structures of formulas (I) to (VII).
[2] The porous coordination network according to [1], in which the metal is at least one selected from Mg, Ca, Mn, Fe, Co, Ni, Cu, Zn, Rh, Ag, Cd, Ir, Pt, and Au.
[3] The porous coordination network according to [1] or [2], in which
   the L2a and the L2b each have a molecular weight of 90 to 1000 and are at least one selected from the following formula (3):
      [Chemical Formula 3]

      R¹-(COOH)ₙ (3)
   where n represents an integer of 2 or more for the L2a, and represents an integer of 3 or more for the L2b, and
   R¹ has at least any one of structures of
   a linear or branched aliphatic group having 2 to 20 carbon atoms,
   an aromatic group having 6 to 72 carbon atoms, and
   a heterocycle having 2 to 60 carbon atoms,
   optionally having an unsaturated bond, optionally having a bond including a heteroatom, and optionally having a substituent.
[4] The porous coordination network according to any one of [1] to [3], in which the porous coordination network is used for guest molecule analysis.
[5] The porous coordination network according to any one of [1] to [4], in which the guest molecule is a medium-sized molecule having a molecular weight of 50 to 7000.
[6] A method for preparing a sample for crystal structure analysis, including:
   providing a sample in which a compound to be analyzed is dissolved in a solvent;
   dispersing the porous coordination network according to any one of [1] to [5] in the sample; and
   incorporating the compound to be analyzed into pores of the porous coordination network.
[7] A method for determining a molecular structure of a compound to be analyzed, including performing crystal structure analysis using a sample for crystal structure analysis obtained by the method for preparing a sample for crystal structure analysis according to [6].

### Advantageous Effects of Invention

The present disclosure has the excellent effect of providing a porous coordination network having a novel structure, a method for preparing a sample for crystal structure analysis, and a method for determining a molecular structure.

### Brief Description of Drawings

Fig. 1 is a diagram showing the coordination environment of PCNI-1 obtained in Example 1;
Fig. 2 is a diagram showing the state of the pores of PCNI-1 obtained in Example 1 when observed from the a-axis.;
Fig. 3 is a diagram showing the coordination environment of PCNI-2 obtained in Example 2;
Fig. 4 is a diagram showing the state of the pores of PCNI-2 obtained in Example 2 when observed from the c-axis;
Fig. 5 is a diagram showing the coordination environment of PCNI-3 obtained in Example 3;
Fig. 6 is a diagram showing the state of the pores of PCNI-3 obtained in Example 3 when observed from the b-axis;
Fig. 7 is a diagram showing the coordination environment of PCNI-4 obtained in Example 4;
Fig. 8 is a diagram showing the state of the pores of PCNI-4 obtained in Example 4 when observed from the b-axis;
Fig. 9 is a diagram showing the coordination environment of PCNII-1 obtained in Example 5;
Fig. 10 is a diagram showing the state of the pores of PCNII-1 obtained in Example 5 when observed from the c-axis;
Fig. 11 is a diagram showing the coordination environment of PCNIII-1 obtained in Example 6;
Fig. 12 is a diagram showing the state of the pores of PCNIII-1 obtained in Example 6 when observed from the a-axis;
Fig. 13 is a diagram showing the coordination environment of PCNIV-1 obtained in Example 7;
Fig. 14 is a diagram showing the state of the pores of PCNIV-1 obtained in Example 7 when observed from the a-axis;
Fig. 15 is a diagram showing the coordination environment of PCNV-1 obtained in Example 8;
Fig. 16 is a diagram showing the state of the pores of PCNV-1 obtained in Example 8 when observed from the c-axis;
Fig. 17 is a diagram showing the coordination environment of PCNVI-1 obtained in Example 9;
Fig. 18 is a diagram showing the state of the pores of PCNVI-1 obtained in Example 9 when observed from the a-axis;
Fig. 19 is a diagram showing the coordination environment of PCNVII-1 obtained in Example 10;
Fig. 20 is a diagram showing the state of the pores of PCNVII-1 obtained in Example 10 when observed from the c-axis;
Fig. 21 is a diagram showing the results of Example 11 in which a guest molecule (spironolactone) was included in PCNI-3 (Example 3). The guest molecule is represented by a ball-and-stick model, and PCN is represented by a stick model (the same applies to the following figures);
Fig. 22 is a diagram showing the results of Example 12 in which a guest molecule (betamethasone) was included in PCNI-3 (Example 3);
Fig. 23 is a diagram showing the results of Example 13 in which a guest molecule (griseofulvin) was included in PCNV-1 (Example 8);
Fig. 24 is a diagram showing the results of Example 14 in which a guest molecule (betamethasone) was included in PCNVII-1 (Example 10);
Fig. 25 is a diagram showing the results of Example 15 in which a guest molecule (ivermectin) was included in the PCN pores of PCNVII-1 (Example 10);
Fig. 26 is a diagram showing the coordination environment of PCNI-5 (Example 16);
Fig. 27 is a diagram showing the state of the pores when PCNI-5 obtained in Example 16 is observed;
Fig. 28 is a diagram showing the results of including a guest molecule (ROY) in the pores of PCNI-5 (Example 16);
Fig. 29 is a diagram showing the coordination environment of PCNVIII-1 (Example 18);
Fig. 30 is a diagram showing the state of the pores when PCNVIII-1 obtained in Example 18 is observed; and
Fig. 31 is a diagram showing the results of includion of a guest molecule (sodium monensin) in a PCNV-1 pore (Example 19).

### Description of Embodiments

An example of an embodiment to which the present disclosure is applied is described below. Other embodiments are also included within the scope of the present disclosure as long as they conform to the spirit of the present disclosure. In addition, the size and ratios of each portion in the following figures are for the convenience of explanation and are not intended to be limiting.

The porous coordination network (hereinafter also referred to as the present PCN) according to the present disclosure has a three-dimensional network structure. This three-dimensional network structure is formed by coordinating ligands, which will be described later, to a metal. In the present PCN, pores capable of encapsulating guest molecules are formed.

The present PCN is represented by any of the following formulas (I) to (VIII). Ligands and metals are combined via coordinate bonds to form a regular structure.

The present PCN has a crystal structure having a unit structure represented by any one of the following formulas (I) to (VIII):

(M²⁺)₄(L1a⁻)₂(L2a²⁻)₃ formula (I)

(M²⁺)₁₁(L1a⁻)₆(L2a²⁻)₈ formula (II)

[(M²⁺)₅(Lia⁻)₄(L2a²⁻)₄]·Y₂ₚ formula (III)

[(M²⁺)₂(L1a)₁(L2a²⁻)₂]·Yₚ formula (IV)

(M²⁺)₂(L1a⁻)₁(L2b³⁻)₁ formula (V)

[(M^{Z+})₃(L1a⁻)₂(L2b³⁻)₂]·Y₂ₚ formula (VI)

[(M²⁺)₈(L1b³⁻)₂(L2a²⁻)₃(L3⁻)₆]·Y₂ₚ formula (VII)

(M²⁺)₅(M³⁺)₁(L1a⁻)₃(L2a²⁻)₅ formula (VIII)

In the formulas (I) to (VIII), M²⁺ is a divalent metal ion, M³⁺ is a trivalent metal ion, and L1a and L1b are tridentate ligands having hexaazaphenalenyl. L1a⁻ is a first ligand ion represented by the following formula (1), or a first ligand ion in which 1 to 12 hydrogen atoms in the aromatic ring of formula (1) are each independently replaced by a group selected from an alkyl group having 1 to 4 carbon atoms, a halogen, a hydroxyl group, and an amino group. 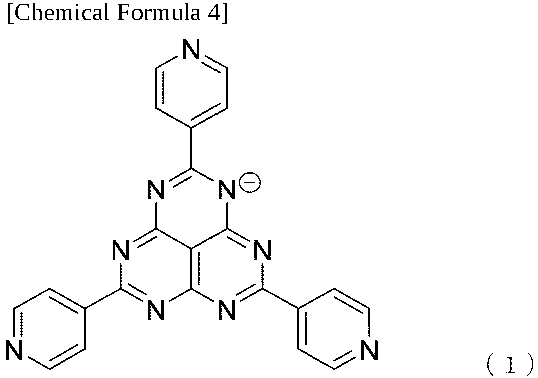 L1b³⁻ is a first ligand ion represented by the following formula (2), or a first ligand ion in which 1 to 12 hydrogen atoms of the aromatic ring of formula (2) are each independently replaced by a group selected from an alkyl group having 1 to 4 carbon atoms, a halogen, a hydroxyl group, and an amino group.

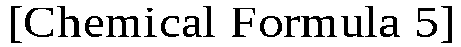

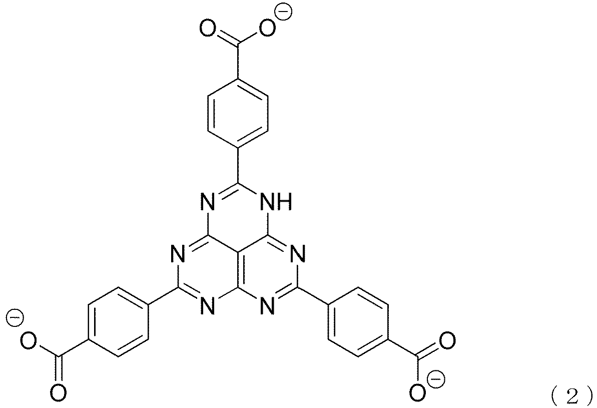

L2a is a bidentate ligand containing two or more carboxy groups. The upper limit of the number of carboxy groups is not particularly limited, but is, for example, 4, with a preferred upper limit of 3 and particularly preferably 2. L2a²⁻ is a second ligand ion in which two of the carboxy groups are coordinated to the metal ion as carboxylate anions.

L2b is a bidentate ligand containing three or more carboxy groups. The upper limit of the number of carboxy groups is not particularly limited, but is, for example, 6, with a preferred upper limit of 4 and particularly preferably 3. L2b³⁻ is a second ligand ion in which three of the carboxy groups are coordinated to the metal ion as carboxylate anions.

L3⁻ is a third ligand ion, which is an anion of triazole or a triazole derivative. The triazole may be either 1,2,3-triazole or 1,2,4-triazole. The triazole derivative refers to a compound having a triazole. For example, the triazole derivative refers to benzotriazole or a compound in which one or more hydrogen atoms of benzotriazole are substituted with, for example, an amino group, a hydroxy group, a methyl group, a nitro group, a halogen group, or a carboxy group. Examples thereof include 4-carboxybenzotriazole, 7-carboxybenzotriazole, benzotriazole butyl ester, 1-hydroxymethylbenzotriazole, and 1-hydroxybenzotriazole.

The present disclosure can provide a PCN with a novel structure by a PCN that combines the specific metal described above with a first ligand and a second ligand, or by a PCN that combines the specific metal described above with a first ligand, a second ligand, and a third ligand. Using a highly symmetric first ligand ion of formula (1) or a first ligand ion of formula (2) can increase the periodicity of the structure and improve the crystallinity. As a result, crystals suitable for structural analysis can be provided.

In addition, using a highly symmetrical first ligand ion of formula (1) and a second ligand ion having two or three carboxylate anions (carboxylate groups); or using a highly symmetrical first ligand ion of formula (2), a second ligand ion having two carboxylate anions (carboxylate groups), and a third ligand ion, forms a metal complex cluster with a divalent metal ion, thereby providing the excellent effect of easily obtaining a porous coordination network having a large pore size and high solvent stability. Further, the PCN is used to form a metal complex cluster with divalent metal ions, thereby allowing the physical and chemical stability of the structure to be increased. That is, due to the physical stability, it is possible to obtain robustness that allows the structure to be maintained if there are large pores. In addition, because of the excellent chemical stability, it is expected to have the effect of increasing solvent stability. The present PCN can be suitably used for guest molecule analysis.

In formulas (I) to (VIII), M²⁺ is a divalent metal ion, and M³⁺ is a trivalent metal ion, and the type is not particularly limited. Preferred examples of metals include at least one selected from Mg, Ca, Mn, Fe, Co, Ni, Cu, Zn, Rh, Ag, Cd, Ir, Pt, and Au. It is possible to use multiple types of metals, but it is preferable to use a single metal from the viewpoint of ease of structural analysis.

The structures of L2a and L2b are not particularly limited as long as they satisfy the above-described conditions. From the viewpoint of easily forming PCN, the molecular weight is preferably 90 to 1000, more preferably 100 to 900, still more preferably 110 to 850, and particularly preferably 116 to 801.

Preferred examples of L2a and L2b include at least one having a molecular weight of 90 to 1000 and selected from the following formula (3).
[Chemical Formula 6]

R¹-(COOH)ₙ (3)

n is an integer of 2 or more for the L2a, the upper limit of which is preferably 4, and more preferably 3. n is an integer of 3 or more for L2b, the upper limit of which is preferably 6, and more preferably 4. R¹ has at least any one of (i) a linear or branched aliphatic group having 2 to 20 carbon atoms, optionally having an unsaturated bond, optionally having a bond including a heteroatom, and optionally having a substituent; (ii) an aromatic group having 6 to 72 carbon atoms, optionally having an unsaturated bond, optionally having a bond including a heteroatom, and optionally having a substituent; and (iii) a heterocycle having 2 to 60 carbon atoms, optionally having an unsaturated bond, optionally having a bond including a heteroatom, and optionally having a substituent. From the viewpoint of the robustness of the PCN structure, it is preferable that the PCN has an aromatic ring, an unsaturated bond, and the like. The more preferred range for the number of carbon atoms in (i) is 2 to 16, and particularly preferred is 2 to 10. The more preferred range for the number of carbon atoms in (ii) is 6 to 60, and particularly preferred is 6 to 55. The more preferred range for the number of carbon atoms in (iii) is 6 to 60, and particularly preferred is 6 to 55. The aliphatic group may be linear or alicyclic.

In addition, preferred examples of the L2a and L2b include compounds having the structure of the following general formula (4). The preferred range of the molecular weight is 90 to 1000, and the more preferred range is as described above. 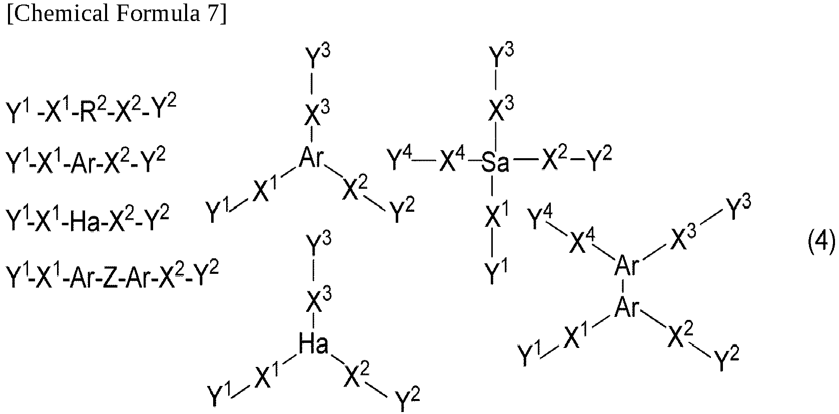 In formula (4), Ar represents a divalent or trivalent aromatic group optionally having a substituent. Ha represents a bond including a heteroatom. Sa represents an atom that forms four bonds in the directions of vertices of a tetrahedron, such as carbon or silicon, or an ion that forms four bonds in the direction of vertices of a tetrahedron, such as ammonium or phosphonium ion. Z is a carbonyl group, a sulfonyl group, an aliphatic group having 1 to 10 carbon atoms optionally having a substituent, or a single bond directly linking Ar to another Ar. X¹ to X⁴ each independently represent a divalent organic group, or a single bond directly linking any of Ar, Ha, or Sa to a carboxy group. Y¹ to Y⁴ each independently represent a monovalent organic group having one or more carboxy groups as a coordinating site or a carboxylic acid. Preferred examples of X¹ to X⁴ include a single bond, a linear or branched aliphatic group having 2 to 10 carbon atoms, an alicyclic aliphatic group having 5 to 15 carbon atoms, an aromatic group having 6 to 24 carbon atoms, and a group having a heterocycle having 2 to 20 carbon atoms, these groups of which optionally have a substituent. More preferred examples of X¹ to X⁴ include a single bond, a linear or branched aliphatic group having 2 to 8 carbon atoms, an alicyclic aliphatic group having 5 to 10 carbon atoms, an aromatic group having 6 to 18 carbon atoms, and a group having a heterocycle having 2 to 15 carbon atoms, these groups of which optionally have a substituent.

Specific examples of L2a include the following compound.

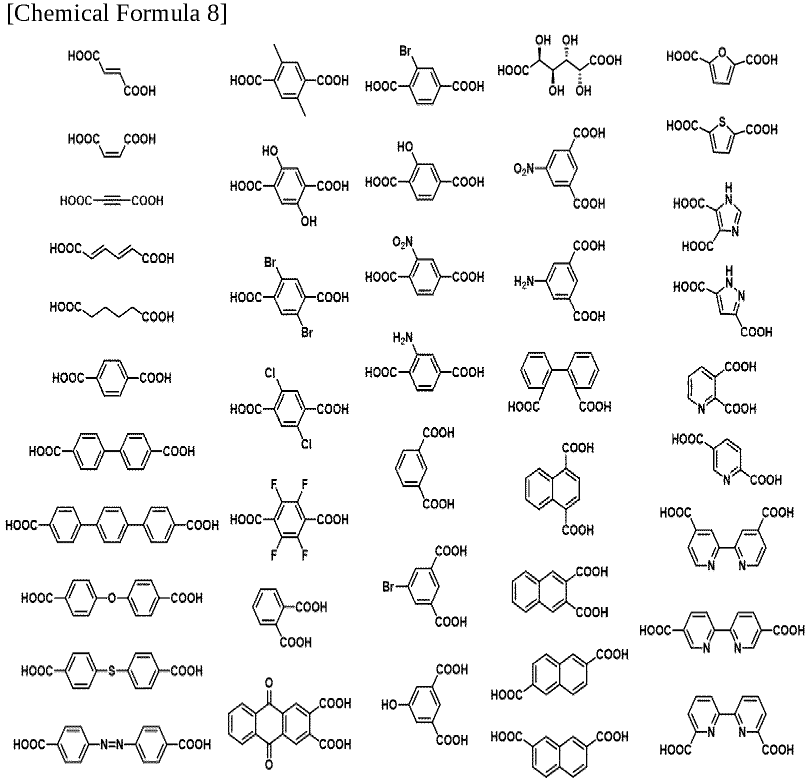

Specific examples of L2b include the following compound.

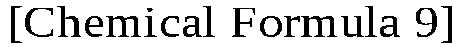

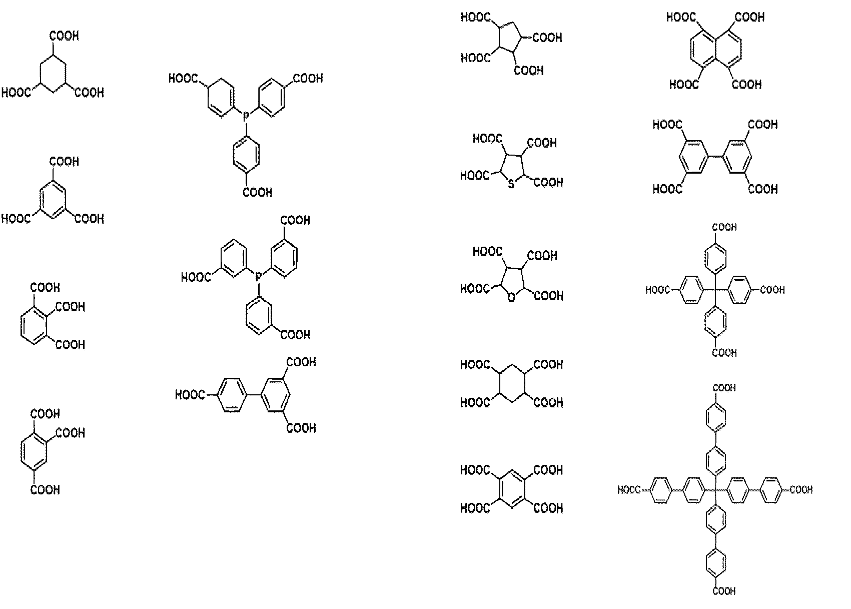

The shape and size of the pores in PCN can be adjusted by changing the ligands. In addition, when the same ligand is used, the shape and size of the pores can be changed by changing the synthesis method.

The pore size of PCN can be understood as the body diagonal distance when the pores not being filled with spheres of the van der Waals radius of the atoms to form the network structure are considered to be cubes. The maximum size of the pores of PCN, which is defined as the short-axis distance when a cut is made at the opening of the pore and the cut is considered to be a rectangle, is not limited, but from the viewpoint of use as a sample for crystal structure analysis, the maximum size is preferably 0.8 nm or more. The maximum size is more preferably 1 nm or more, and even more preferably 1 to 5.0 nm. The opening diameter of the pores is determined primarily by the type of first ligand. Making the opening size of the pores 1.5 nm or more allows the compounds with molecular weights of, for example, about 100 to 3,000 to be selectively incorporated into the hollow space, and allows a variety of applications including crystal structure analysis.

Y is not particularly limited, but from the viewpoint of being present in the PCN pores, the molecular weight is preferably 1 to 600, and more preferably 6 to 500. In addition, from the viewpoint of the interaction between Y and the inclusion guest, it is preferable to have an alkali metal, an alkaline earth metal, or an organic cation.

Specific examples of Y include inorganic cations such as Li⁺, Na⁺, K⁺, Rb⁺, Cs⁺, Be²⁺, Mg²⁺, Ca²⁺, Sr²⁺, and Ba²⁺, and cationic components of organic salts, such as cationic components of organic ammonium salts, organic oxonium salts, organic arsonium salts, organic sulfonium salts, organic bromonium salts, organic phosphonium salts, and organic iodonium salts.

A solvent may be coordinated to the PCN. There are no limitations on the type of solvent that may be coordinated to the PCN, but polar solvents are preferable from the viewpoint of forming hydrogen bond sites. Suitable examples of the polar solvent include: alcohols such as water, methanol, ethanol, and isopropanol; glycols such as ethylene glycol and polyethylene glycol; ester-based solvents such as ethyl acetate, butyl acetate, and propylene glycol monomethyl ether acetate; ketone-based solvents such as acetone, ethyl methyl ketone, and cyclohexanone; ether-based solvents such as diethyl ether, diphenyl ether, tetrahydrofuran, 1,4-dioxane, cyclopentyl methyl ether, and methyl tert-butyl ether; N,N'-dimethylformamide, dimethyl sulfoxide, and N-methylpyrrolidone. These may be used singly or in combination of two or more.

The axis of the 4-carboxybenzyl group used as L1b in formula (VII) rotates freely relative to the hexaazaphenalenyl, providing excellent symmetry. **In** the PCN in which the oxygen of the carboxylate of this 4-carboxybenzyl group is coordinated with a metal, the free rotation of the axis of the 4-carboxybenzyl group is not hindered. The 4-pyridinyl group used as L1a⁻ in formulas (I) to (VI) also rotates freely relative to the hexaazaphenalenyl, providing excellent symmetry.

The PCN represented by formula (I) has a crystal structure having a unit structure of four metals, two first ligand ions, and three second ligand ions. A solvent may further be coordinated to the PCN. Suitable examples of the structural unit of formula (I) include (Mn²⁺)₄(TPHAP⁻)₂(BDC²⁻)₃, (Mn²⁺)₄(TPHAP⁻)₂(NDC²⁻)₃, (Mn²⁺)₄(TPHAP⁻)₂(BPDC²⁻)₃, (Fe²⁺)₄(TPHAP⁻)₂(BDC²⁻)₃, (Fe²⁺)₄(TPHAP⁻)₂(NDC²⁻)₃, (Fe²⁺)₄(TPHAP⁻)₂(BPDC²⁻)₃, (Co²⁺)₄(TPHAP⁻)₂(BDC²⁻)₃, (Co²⁺)₄(TPHAP⁻)₂(NDC²⁻)₃, (Co²⁺)₄(TPHAP⁻)₂(BPDC²⁻)₃, (Ni²⁺)₄(TPHAP⁻)₂(BDC²⁻)₃, (Ni²⁺)₄( TPHAP⁻)₂(NDC²⁻)₃, (Ni²⁺)₄(TPHAP⁻)₂(BPDC²⁻)₃, (Cu²⁺)₄(TPHAP⁻)₂(BDC²⁻)₃, (Cu²⁺)₄(TPHAP⁻)₂(NDC²⁻)₃, (Cu²⁺)₄(TPHAP⁻)₂(BPDC²⁻)₃, (Zn²⁺)₄(TPHAP⁻)₂(BDC²⁻)₃, (Zn²⁺)₄(TPHAP⁻)₂(NDC²⁻)₃, (Zn²⁺)₄(TPHAP⁻)₂(BPDC²⁻)₃, (Cd²⁺)₄(TPHAP⁻)₂(BDC²⁻)₃, (Cd²⁺)₄(TPHAP⁻)₂(NDC²⁻)₃, and (Cd²⁺)₄(TPHAP⁻)₂(BPDC²⁻)₃. BDC²⁻is the anion of terephthalic acid, NDC²⁻ is the anion of 2,6-naphthalenedicarboxylic acid, and BPDC²⁻ is the anion of biphenyl-4,4'-dicarboxylic acid. TPHAP⁻ is the first ligand ion of the above formula (1).

The PCN represented by formula (II) has a crystal structure having a unit structure of 11 metals, 6 first ligand ions, and 8 second ligand ions. A solvent may further be coordinated. Suitable examples of the unit structure of formula (II) include (Mn²⁺)₁₁(TPHAP⁻)₈(MA²⁻)₈, (Fe²⁺)₁₁(TPHAP⁻)₆(MA²⁻)₈, (Co²⁺)₁₁(TPHAP⁻)₆(MA²⁻)₈, (Ni²⁺)₁₁(TPHAP⁻)₆(MA²⁻)₈, (Cu²⁺)₁₁(TPHAP⁻)₆(MA²⁻)₈, (Zn²⁺)₁₁(TPHAP⁻)₆(MA²⁻)₈, and (Cd²⁺)₁₁(TPHAP⁻)₆(MA²⁻)₈. MA²⁻represents the anion of trans,trans-muconic acid.

The PCN represented by formula (III) has a crystal structure having a unit structure of five metals, four first ligand ions, and four second ligand ions. Y is a counter ion. Further, a solvent may be coordinated. Suitable examples of the unit structure include (Mn²⁺)₅(TPHAP⁻)₄(BPDC²⁻)₄·Y₂ₚ, (Fe²⁺)₅(TPHAP⁻)₄(BPDC²⁻)₄·Y₂, (Co²⁺)₅(TPHAP⁻)₄(BPDC²⁻)₄·Y₂, (Ni²⁺)₅(TPHAP⁻)₄(BPDC²⁻)₄·Y₂ₚ, (Cu²⁺)₅(TPHAP⁻)₄(BPDC²⁻)₄·Y₂ₚ, (Zn²⁺)₅(TPHAP⁻)₄(BPDC²⁻)₄·Y₂ₚ, and (Cd²⁺)₅(TPHAP⁻)₄(BPDC²⁻)₄·Y₂ₚ. Y is a cation present as a counter ion. Specific examples of cations include Li⁺, Na⁺, K⁺, Rb⁺, Mg²⁺, Ca²⁺, Sr²⁺, tetra-n-butylammonium (TBA⁺), and dimethylammonium (DMA⁺). The same applies to suitable Y in (IV), (VI), and (VII) described below.

The PCN represented by formula (IV) has a crystal structure having a unit structure of two metals, one first ligand ion, and two second ligand ions. Y is a counter ion. Further, a solvent may be coordinated. Preferred examples of the unit structure include (Mn²⁺)₂(TPHAP⁻)₁(DHC²⁻)₂·Yₚ, (Fe²⁺)₂(TPHAP⁻)₁(DHC²⁻)₂·Yₚ, (Co²⁺)₂(TPHAP⁻)₁(DHC²¯)₂·Yₚ, (Ni²⁺)₂(TPHAP⁻)₁(DHC²⁻)₂·Yₚ, (Cu²⁺)₂(TPHAP⁻)₁(DHC²⁻)₂·Yₚ, (Zn²⁺)₂(TPHAP⁻)₁(DHC²⁻)₂·Yₚ, and (Cd²⁺)₂(TPHAP⁻)₁(DHC²⁻)₂·Yₚ. DHC²⁻ represents the anion of 2,5-dihydroxyterephthalic acid. In addition, Y is a cation that exists as a counter ion.

The PCN represented by formula (V) has a crystal structure having a unit structure of two metals, one first ligand ion, and one second ligand ion. Further, a solvent may be coordinated. Preferred examples of the unit structure include (Mn²⁺)₂(TPHAP⁻)(TCBM³⁻), (Fe²⁺)₂(TPHAP⁻)(TCBM³⁻), (Co²⁺)₂(TPHAP⁻)(TCBM³⁻), (Ni²⁺)₂(TPHAP⁻)(TCBM³⁻), (Cu²⁺)₂(TPHAP⁻)(TCBM³⁻), (Zn²⁺)₂(TPHAP⁻)(TCBM³⁻), and (Cd²⁺)₂(TPHAP⁻)(TCBM³⁻). TCBM³⁻ is the anion of 5',5"-bis(4-carboxyphenyl)-2',2",4',4",6',6"-hexamethylene-[1,1':3',1":3",1"'-quaterphenyl]-4,4"'-dicarboxylic acid.

The PCN represented by formula (VI) has a crystal structure having a unit structure of three metals, two first ligand ions, and one second ligand. Further, Y is a counter ion. However, the charge of the metal and the second ligand is balanced in formula (VI), which thus may also include compounds that do not include a first ligand. From a macroscopic viewpoint, it is known that the composition taking into account defects is [(M²⁺)₃(Lia⁻)_{(4+2x)/3}(L2b³⁻)₂]·Y_{(4+2x)/3} (0 < x ≤ 1). Further, a solvent may be coordinated. Suitable examples of the unit structure include [(Mn²⁺)₃(TPHAP⁻)₂(BTCA³⁻ )₂]·Y₂ₚ, [(Mn²⁺)₃(TPHAP⁻)₂(BTCA³⁻)₂]·Y₂ₚ, [(Fe²⁺)₃(TPHAP⁻)₂(BTCA³⁻)₂]·Y₂ₚ, [(Co²⁺)₃(TPHAP⁻)₂(BTCA³⁻)₂]·Y₂ₚ, (Ni²⁺)₃(TPHAP⁻)₂(BTCA³⁻)₂]·Y₂ₚ, [(Cu²⁺)₃(TPHAP⁻)₂(BTCA³⁻)₂]·Y₂ₚ, [(Zn²⁺)₃(TPHAP⁻)₂(BTCA³⁻)₂]·Y₂ₚ, and [(Cd²⁺)₃(TPHAP⁻)₂(BTCA³⁻)₂]·Y₂ₚ. BTCA³⁻ is the anion of [1,1'-biphenyl]-3,4',5-tricarboxylic acid.

The PCN represented by formula (VII) has a crystal structure having a unit structure of two metals, two first ligand ions, three second ligand ions, and six tertiary ligand ions. Y is a counter ion. In addition, a solvent may be coordinated. Suitable examples of the unit structure include (Mn²⁺)₈(HTCHAP³⁻)₂(TDC²⁻)₃(TAZ⁻)₆·Y₂ₚ, (Fe²⁺)₈(HTCHAP³⁻)₂(TDC²⁻)₃(TAZ⁻)₆·Y₂ₚ, (Ni²⁺)₈(HTCHAP³⁻)₂(TDC²⁻)₃(TAZ⁻)₆·Y₂ₚ, (Co²⁺)₈(HTCHAP³⁻)₂(TDC²⁻)₃(TAZ-)⁶·Y₂ₚ, (Zn²⁺)₈(HTCHAP³⁻)₂(TDC²⁻)₃(TAZ⁻)₆·Y₂ₚ, and (Cd²⁺)₈(HTCHAP³⁻)₂(TDC²⁻)₃(TAZ⁻)₆·Y₂ₚ. TDB²⁻is the anion of 2,5-(thiophenediyl)dibenzoic acid, and TDC²⁻ is the anion of 2,5-thiophenedicarboxylic acid, and TAZ- is the anion of 1,2,3-triazole. In addition, HTCHA^{p3-} is the first ligand ion of the above formula (2).

The PCN represented by formula (VIII) has a crystal structure having a unit structure of six metals (including five divalent metal ions and one trivalent metal ion), three first ligand ions, and five second ligand ions. In addition, a solvent may be coordinated. Suitable examples of the unit structure include (Co²⁺)₅(Co³⁺)₁(TPHAP⁻)₃(FDCA²⁻)ₛ, (Mn²⁺)₅(Mn³⁺)₁(TPHAP⁻)₃(FDCA²⁻)₅, and (Fe²⁺)₅(Fe³⁺)₁(TPHAP⁻)₃(FDCA^{Z-})ₛ. FDCA²⁻ is the anion of 2,5-furandicarboxylic acid.

The shape of the pores in PCN varies depending on the raw material molecules used. The size of the pore opening can be easily adjusted depending on the type of ligand and metal. For the analysis of medium-sized molecules, a PCN with as large an opening size as possible is preferable.

Solvent molecules such as DMF may be coordinated to the metal. The bond between the solvent molecules incorporated into the unit crystal and the metal is preferably weaker than the coordinate bond between the metal and the first ligand, and between the metal and the second ligand, or between the second ligand and the third ligand. Incorporating solvent molecules into the crystal structure units of the PCN is expected to exhibit the effect of reducing structural fluctuations when analyzing guest molecules to improve the accuracy of the analysis. In addition, it is possible to replace the solvent depending on the guest molecules being incorporated, and it is expected to increase the number of subjects to be analyzed. Further, these solvent molecules inside the pores can be confirmed through analysis.

The present PCN is suitable as a crystal for analyzing the crystal structure of high molecular weight guest molecules, such as pharmaceuticals and peptides. In addition, forming the crystal having an organic solvent as a ligand allows the position of the guest molecule relative to the solvent molecule to be analyzed. Further, making the bond between this organic solvent and the metal weaker than the coordinate bonds between the first ligand and the metal, and the coordinate bonds between the second ligand, or the second ligand and the third ligand, and the metal, thereby allowing the freedom of replacement with other solvent molecules to be increased. Therefore, it is possible to adjust the environmental field of the pores of the PCN depending on the guest molecule. These effects are expected to improve the accuracy of crystal structure analysis.

Further, the present PCN can provide various PCNs with new structures by combining a metal, a first ligand, and a second ligand, or by combining a metal, a first ligand, a second ligand, and a third ligand. In addition, as shown in the examples described below, it is possible to change the structure of the PCN obtained by the synthetic method in the same combination. For this reason, for example, multiple test tubes including different types of PCN and a solvent are provided, a guest molecule is added thereto, and a PCN that is compatible with the guest molecule can be easily screened. As a result, it is expected to easily analyze the structures of medium-sized molecules, which have been particularly difficult to analyze until now.

The examples of use for analysis are described above, but it is also suitable for applications in substance purification processes and as a reaction field in closed spaces. In addition, the surface area is significantly larger than that of zeolites, and thus the application to an adsorbent or purification purposes is expected. Further, the application to sensing organic substances, i.e. a chemical sensor material is possible. In addition, the application to drug delivery systems that release adsorbed guest molecules in response to external stimuli such as UV light is expected.

### (Production method)

Then, an example of a method for producing the PCN will be described. However, the method for producing the PCN according to the present disclosure is not limited to the following method.

First, a metal or metal compound, ligands (first ligand/second ligand, or first ligand/second ligand/tertiary ligand), and a solvent are placed in a container and heated, and synthesis can be performed using the solvothermal method. This allows the PCN having pores to be produced through a self-organizing one-step reaction. After synthesis of the PCN, crystals can be obtained by thorough washing. Instead of the solvothermal method, the PCN may be synthesized by the solution diffusion method, electrochemical synthesis method, microwave irradiation method, mechanochemical synthesis method, or ultrasonic synthesis method. Various PCNs can be synthesized by changing the solvent and the concentration thereof, the molar ratio between metal and ligand, temperature, and metal ion.

The molar ratio between the first ligand and the metal used is 1 : 1 to 1 : 10, preferably 1 : 1 to 1 : 5, and more preferably 1 : 1 to 1 : 3. The molar ratio between the second ligand (the second and third ligands, if the third ligand is used) and the metal is 1 : 1 to 1 : 7, preferably 1 : 1 to 1 : 5, and more preferably 1 : 1 to 1 : 3.

The organic solvent to be used is not particularly limited as long as it can uniformly dissolve the first ligand and the metal. Examples of the solvent include aromatic hydrocarbons such as benzene, toluene, xylene, chlorobenzene, 1,2-dichlorobenzene, mesitylene, and nitrobenzene; sulfoxides such as dimethyl sulfoxide (DMSO); amides such as N,N-dimethylformamide, N,N-dimethylacetamide, and N-methyl-2-pyrrolidone; ethers such as tetrahydrofuran, 1,2-dimethoxyethane, and 1,4-dioxane; alcohols such as methanol, ethanol, and isopropyl alcohol; and ketones such as acetone and methyl ethyl ketone. The solvent may be used singly or in combination of two or more.

Among these, amide-based reaction solvents are preferred as the reaction solvent, because of allowing the porous coordination network of the present disclosure to be synthesized in higher yield, and DMF, DMA, NMP, and the like are particularly suitable.

The precipitated PCN can be collected by filtration. The structure of the PCN can be confirmed by elemental analysis, IR, UV absorption spectroscopy, visible light absorption spectroscopy, single crystal X-ray structure analysis, and the like.

### (Method of preparing sample for crystal structure analysis)

Then, a method for preparing a sample for crystal structure analysis according to the present embodiment will be described. The preparation of the sample for crystal structure analysis involves incorporating a compound to be analyzed (guest molecule) into the pores of the PCN to provide a sample for structural analysis of the compound.

First, a sample in which the compound to be analyzed is dissolved in a solvent is provided, and the PCN is dispersed in the sample. The sample is prepared by, for example, a step of incorporating the compound to be analyzed into the pores of the PCN and removing the solvent.

A method for bringing a mixture including a compound to be analyzed into contact with the PCN and incorporating molecules of the compound to be analyzed into the pores of the PCN can be performed by a known method.

The compound to be analyzed is not particularly limited, as long as it is of a size that can enter the pores of the PCN. The application is possible to a wide variety of compounds, including low molecular weight compounds, medium-sized molecule compounds such as pharmaceuticals, and chiral compounds. For example, molecules having a molecular weight of about 50 to 7000 can be analyzed. Suitable is a compound having a molecular weight of preferably 5000 or less, more preferably 480 to 4000, and particularly preferably 500 to 3000.

The method for contacting the PCN with the compound to be analyzed is not particularly limited. For example, for a method for preparing a solution of the compound to be analyzed and bringing the PCN to be brought into contact with this solution, or in a case where the compound to be analyzed is a liquid or gas, the molecules of the compound to be analyzed can be incorporated in the pores of the PCN by directly contacting the PCN with the compound to be analyzed. Among these, the method of preparing a solution of the compound to be analyzed and contacting the PCN with this solution is preferable because it is easier to obtain a better quality sample for crystal structure analysis.

In addition, when using a solution including the compound to be analyzed or when the compound to be analyzed is liquid, the contact operation can be performed by a method for immersing the single crystal in a solution including the compound to be analyzed, by a method for packing the single crystal into a capillary and then passing a solution including the compound to be analyzed through the capillary, or the like.

The solvent of the solution including the compound to be analyzed may be any solvent that does not dissolve the single crystal used and dissolves the compound to be analyzed. Examples of the solvent include aromatic hydrocarbons such as benzene, toluene, xylene, chlorobenzene, 1,2-dichlorobenzene, nitrobenzene, and mesitylene; aliphatic hydrocarbons such as n-butane, n-pentane, n-hexane, and n-heptane; alicyclic hydrocarbons such as cyclopentane, cyclohexane, and cycloheptane; nitriles such as acetonitrile and benzonitrile; sulfoxides such as dimethyl sulfoxide (DMSO); amides such as N,N-dimethylformamide and n-methylpyrrolidone; ethers such as diethyl ether, tetrahydrofuran, 1,2-dimethoxyethane, and 1,4-dioxane; alcohols such as methanol, ethanol, and isopropyl alcohol; ketones such as acetone, methyl ethyl ketone, and cyclohexanone; halogenated hydrocarbons such as dichloromethane, chloroform, carbon tetrachloride, and 1,2-dichloroethane; esters such as methyl acetate, ethyl acetate, ethyl lactate, and ethyl propionate; and water. The solvent can be used singly or in combination of two or more.

The contact time between the compound to be analyzed and the PCN is not particularly limited, but is typically from a few minutes to a few months. Depending on the compound, the contact time can be shortened or lengthened as appropriate. The contact temperature is not particularly limited, and can be in the range of, for example, -30 to 200°C. It is preferable to perform the contact at a temperature at which the compound to be analyzed can be easily handled.

A sample for crystal structure analysis has molecules of the compound to be analyzed incorporated into the pores of the PCN. Due to the size of the pore opening, the compound to be analyzed is regularly disposed within the pores. Being regularly disposed in this case means that the molecules of the compound to be analyzed are incorporated into the pores to an extent that the structure thereof can be determined by crystal structure analysis.

The sample for crystal structure analysis may be only capable of determining the molecular structure of the compound to be analyzed, and it is not necessary for the molecules of the compound to be analyzed to be incorporated into all of the pores of the PCN. The molecular structure of the compound to be analyzed is determined by performing crystal structure analysis of the compound to be analyzed using the sample for crystal structure analysis. Structural analysis can utilize X-ray diffraction, neutron diffraction, and the like. Performing crystal structure analysis using a sample for crystal structure analysis obtained by the method for preparing a sample for crystal structure analysis can determine the molecular structure of the compound to be analyzed. The present disclosure allows crystal structure analysis by using a sample for crystal structure analysis obtained by the above-described method for preparing a sample for crystal structure analysis.

### <<Examples>>

The present disclosure will be described in more detail below with reference to examples, but the present disclosure is not limited to the following examples.

### [Physical property evaluation]

### (Solvent stability)

A drop of a solvent including a PCN was placed into a glass vial. Subsequently, approximately 2 cm³ of the solvent to be tested for stability was added, and the supernatant was then immediately removed by decantation. This operation was performed a total of three times, after which the solvent was added and the mixture was allowed to stand at room temperature. After leaving the mixture to stand for several days, an optical microscope was used to check whether the crystalline shape of the PCN had changed.

### (Single crystal X-ray structure analysis)

The PCN that underwent guest inclusion was subjected to single crystal X-ray diffraction measurement using Varimax Saturn, manufactured by Rigaku Corporation, or Synergy-R/DWTI APEX II, manufactured by Rigaku Corporation, or a synchrotron KEK-BL5A.

### [Example 1]

5 mg (0.03 mmol) of terephthalic acid (H₂BDC), 6.4 mg (0.01 mmol) of tetra-n-butylammonium 2,5,8-tri(4'-pyridyl)1,3,4,6,7,9-hexaazaphenalenyl (TBA⁺TPHAP⁻), and 6.6 mg (0.03 mmol) of cobalt bromide were heated with 2 cm³ of DMF in a vial at 80°C for 24 hours to provide a square reddish purple PCN single crystal (PCNI-1).

The resulting reddish purple single crystal PCNI-1 was identified by X-ray structural analysis, and was confirmed to be a PCN having a unit structure represented by (Co²⁺)₄(TPHAP⁻)₂(BDC²⁻)₃.

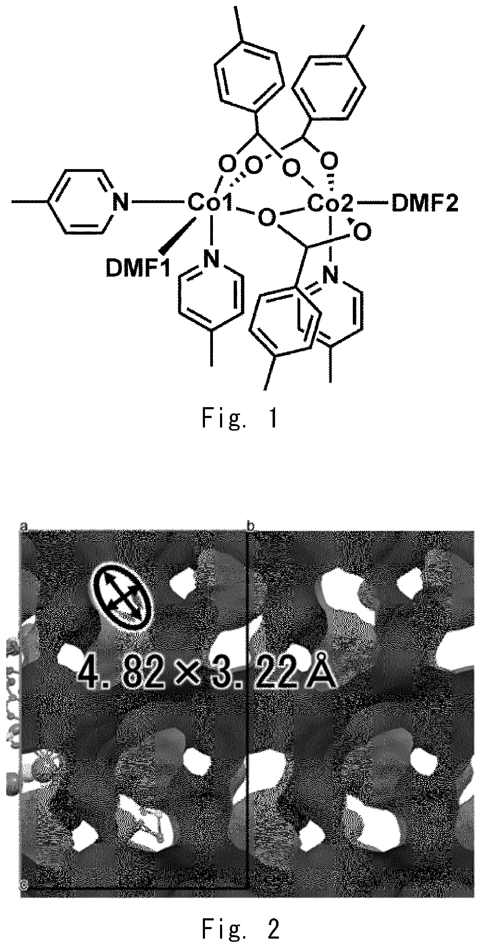

The results of X-ray structural analysis of PCNI-1 are shown in Table 1, Fig. 1 and Fig. 2. The numbers in parentheses in Table 1 indicate statistical errors for the numbers in the table (the same applies to subsequent tables). In addition, Fig. 2 is a view showing the size of pores obtained by simulating atomic coordinate information obtained from X-ray structural analysis with Mercury (manufactured by The Cambridge Crystallographic Data Centre) for a solvent-penetrable surface obtained by adding a probe radius of 1.2 Å to the van der Waals radius. The arrow in the figure indicates the long axis of the pore and the opening size perpendicular to the long axis. In addition, the numbers in the figure indicate the values of the long axis and the opening size (unit: Å). The same applies to subsequent figures.

### [Table 1]

**Table 1**

| Name | PCNI-1 |
|---|---|
| Temperature/K | 173.15 |
| Crystal system | Monoclinic |
| Space group | P2₁/c |
| a/Å | 14.0080 (7) |
| b/Å | 17.6494(7) |
| c/Å | 28.6715(11) |
| *α*/° | 90 |
| *β*/° | 103.259(4) |
| *γ*/° | 90 |
| Volume/Å³ | 6899.6(5) |
| Z | 4 |
| Crystal size/mm³ | 0.115 × 0.043 × 0.025 |
| Radiation | Mo K*α* (*λ* = 0.71073) |
| Independent reflections | 15916 [Rᵢₙₜ = 0.3285, R_{sigma} = 0.3239] |
| Goodness-of-fit on F² | 1.199 |

PCNI-1 is formed based on a cobalt dinuclear cluster including three TPHAP-derived pyridinyl groups, three BDC-derived carboxylic acids, and two DMFs, as shown in Fig. 1. The PCN of Example 1 has pores as shown in Fig. 2, with a maximum opening size of 4.82 × 3.22 Å.

### [Example 2]

5 mg (0.03 mmol) of terephthalic acid (H₂BDC), 6.4 mg (0.01 mmol) of tetra-n-butylammonium 2,5,8-tri(4'-pyridyl)1,3,4,6,7,9-hexaazaphenalenyl (TBA⁺TPHAP⁻), and 6.6 mg (0.03 mmol) of cobalt bromide were heated with 2 cm³ of DMF at 90°C in an autoclave for 24 hours to provide hexagonal reddish purple PCNI-2 single crystals.

The resulting reddish purple single crystal PCNI-2 was identified by X-ray structural analysis and confirmed to be a PCN having a unit structure represented by (Co²⁺)₄(TPHAP⁻)₂(BDC²⁻)₃.

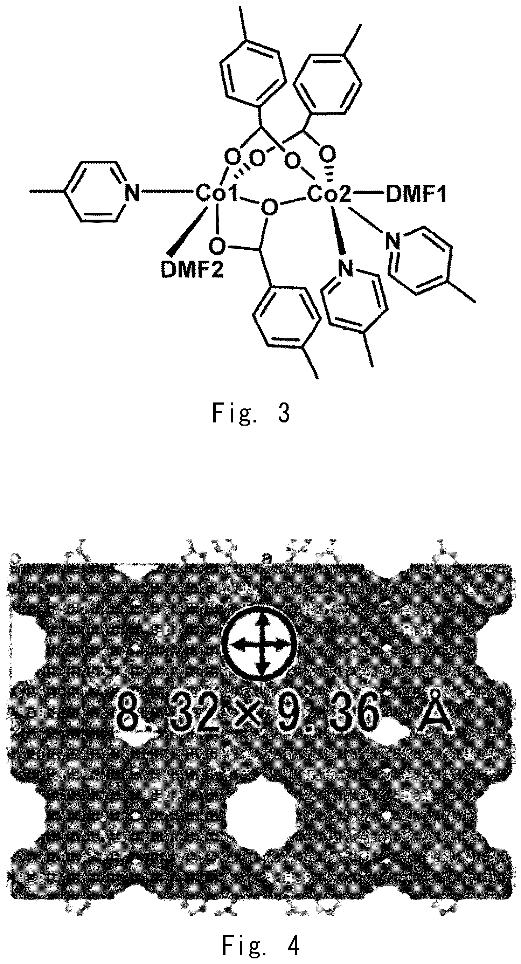

The results of X-ray structural analysis of PCNI-2 are shown in Table 2, Fig. 3, and Fig 4.

### [Table 2]

**Table 2**

| Name | PCNI-2 |
|---|---|
| Temperature/K | 173.15 |
| Crystal system | monoclinic |
| Space group | P2/c |
| a/Å | 30.7062(10) |
| b/Å | 20.5493 (5) |
| c/Å | 22.6908(9) |
| *α*/° | 90 |
| *β*/° | 93.221(3) |
| *γ*/° | 90 |
| Volume/Å³ | 14295.1(8) |
| Z | 4 |
| Crystal size/mm³ | 0.223 × 0.135 × 0.094 |
| Radiation | Mo K*α* (*λ* = 0.71073) |
| Independent reflections | 33005 [Rᵢₙₜ = 0.2634, R_{sigma} = 0.2216] |
| Goodness-of-fit on F² | 1.601 |
| Final R indexes [I>=2σ (I)] | R₁ = 0.2512, wR₂ = 0.5676 |

In PCNI-2, a unit structure is formed based on a cobalt dinuclear cluster formed by the coordination of three TPHAP-derived pyridinyl groups, three BDC-derived carboxyl groups, and two solvents, as shown in Fig. 3. The PCN according to Example 2 had a pore size of 8.32 × 9.36 Å as shown in Fig. 4.

### [Example 3]

65 mg (0.3 mmol) of 2,6-naphthalenedicarboxylic acid (H₂NDC), 128 mg (0.2 mmol) of tetra-n-butylammonium 2,5,8-tri(4'-pyridyl)1,3,4,6,7,9-hexaazaphenalenyl (TBA⁺TPHAP⁻), and 87.5 mg (0.4 mmol) of cobalt bromide were heated in a mixed solvent of 30 cm³ of dehydrated DMF and 15 cm³ of dehydrated acetonitrile (MeCN) at 100°C in a pressure vessel for 72 hours to provide reddish purple single crystals of PCNI-3.

The resulting reddish purple single crystal PCNI-3 was identified by X-ray structural analysis and confirmed to be a PCN having a structural unit represented by (Co²⁺)₄(TPHAP⁻)₂(NDC²⁻)₃.

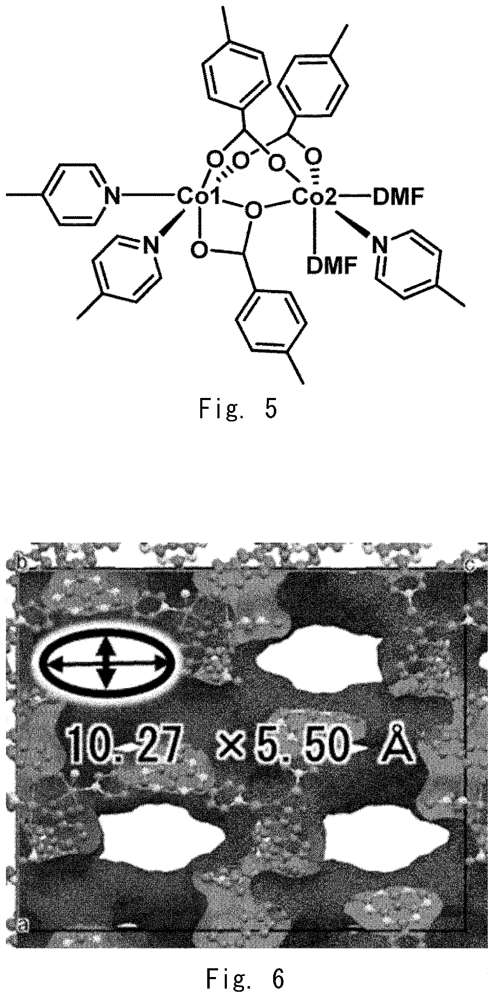

The results of X-ray structural analysis of PCNI-3 are shown in Table 3, Fig. 5, and Fig 6.

### [Table 3]

**Table 3**

| Name | PCNI-3 |
|---|---|
| Temperature/K | 95 |
| Crystal system | Orthorhombic |
| Space group | Pbca |
| a/Å | 29. 216(6) |
| b/Å | 20.741(8) |
| c/Å | 36.290(5) |
| *α*/° | 90 |
| *β*/° | 90 |
| *γ*/° | 90 |
| Volume/Å³ | 21991(10) |
| Z | 8 |
| Crystal size/mm³ | 0.259 × 0.241 × 0.143 |
| Radiation | 0.75Å (KEK BL-5A) |
| Independent reflections | 41733 [Rᵢₙₜ = 0.0465, R_{sigma} = 0.0219] |
| Goodness-of-fit on F² | 1.395 |
| Final R indexes [I>=2σ (I)] | R₁ = 0.1091, wR₂ = 0.3335 |

PCNI-3 has a structure including a cobalt dinuclear cluster obtained by the coordination of three TPHAP-derived pyridinyl groups, three NDC-derived carboxyl groups, and two DMFs, as shown in Fig. 5. As shown in Fig. 6, PCNI-3 has a pore of 5.50 × 10.27 Å.

### [Example 4]

6.5 mg (0.03 mmol) of 2,6-naphthalenedicarboxylic acid (H₂NDC), 6.4 mg (0.01 mmol) of tetra-n-butylammonium 2,5,8-tri(4'-pyridyl)1,3,4,6,7,9-hexaazaphenalenyl (TBA⁺TPHAP⁻), and 6.6 mg (0.03 mmol) of cobalt bromide were heated with a mixed solvent of 2 cm³ of DMF and 0.1 cm³ of methanol (MeOH) in a vial at 80°C for 24 hours to provide square orange PCN single crystals (PCNI-4).

The resulting reddish purple single crystal PCNI-4 was identified by X-ray structural analysis and confirmed to be a PCN having a structural unit represented by (Co²⁺)₄(TPHAP⁻)₂(NDC²⁻)₃.

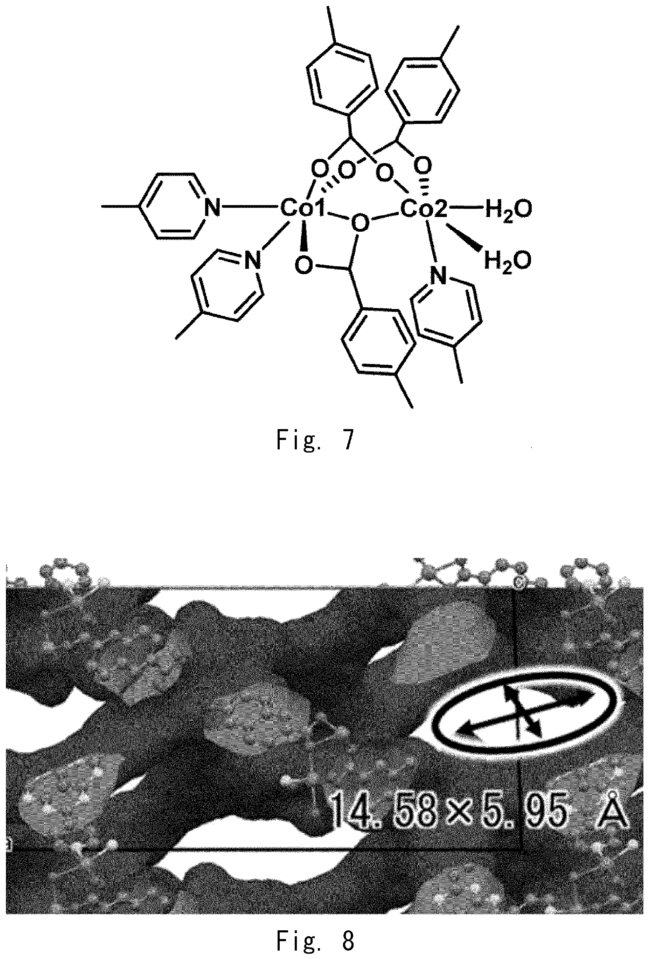

The results of X-ray structural analysis of PCNI-4 are shown in Table 4, Fig. 7, and Fig. 8.

### [Table 4]

**Table 4**

| Name | PCNI-4 |
|---|---|
| Temperature/K | 173.15 |
| Crystal system | Monoclinic |
| Space group | P2₁/n |
| a/Å | 16.6632(11) |
| b/Å | 19.1759(10) |
| c/Å | 33.2438(9) |
| *α*/° | 90 |
| *β*/° | 88.187(4) |
| *γ*/° | 90 |
| Volume/Å³ | 10617.1(9) |
| Z | 4 |
| Crystal size/mm³ | 0.126 × 0.079 × 0.036 |
| Radiation | Mo K*α* ( *λ* = 0.71073) |
| Independent reflections | 24460 [Rᵢₙₜ = 0.1908, R_{sigma} = 0.1798] |
| Goodness-of-fit on F² | 1.483 |
| Final R indexes [I>=2σ (I)] | R₁ = 0.1895, wR₂ = 0.4687 |

PCNI-4 has a unit structure in which three TPHAP-derived pyridyl groups and three NDC-derived carboxyl groups are formed from a cobalt dinuclear cluster, as shown in Fig. 7. PCNI-4 has pores as shown in Fig. 8, with a maximum opening size of 14.58 × 5.95 Å.

### [Example 5]

4.3 mg (0.03 mmol) of trans,trans-muconic acid (H₂MA), 6.4 mg (0.01 mmol) of tetra-n-butylammonium 2,5,8-tri(4'-pyridyl)1,3,4,6,7,9-hexaazaphenalenyl (TBA⁺TPHAP⁻), and 6.6 mg (0.03 mmol) of cobalt bromide were heated with 2 cm³ of DMF in a vial at 80°C for 24 hours to provide tetrahedral orange PCN single crystals (PCNII-1).

The resulting orange single crystal PCNII-1 was identified by X-ray structural analysis and confirmed to be a PCN having a structural unit represented by (Co²⁺)₁₁(TPHAP⁻)₆(MA²⁻)₈.

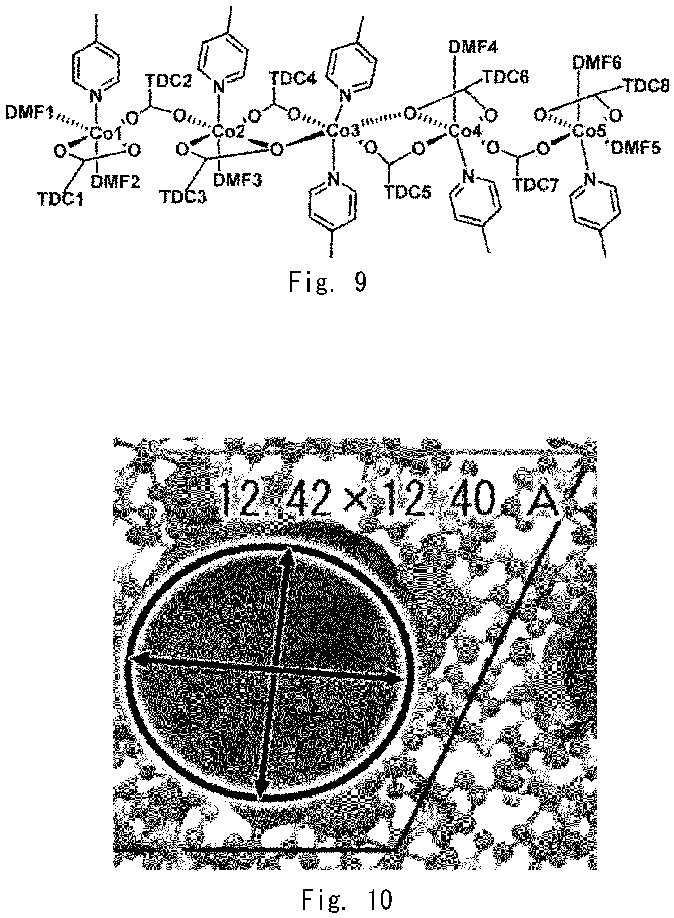

The results of the X-ray structural analysis of PCNII-1 are shown in Table 5, Fig. 9, and Fig. 10.

### [Table 5]

**Table 5**

| Name | PCNII-1 |
|---|---|
| Temperature/K | 95.15 |
| Crystal system | Triclinic |
| Space group | P-1 |
| a/Å | 19.5272(6) |
| b/Å | 19.8546(6) |
| c/Å | 19.9971(5) |
| *α*/° | 109.146(3) |
| *β*/° | 104.885(3) |
| *γ*/° | 108.800(3) |
| Volume/Å³ | 6338.0(4) |
| Z | 2 |
| Crystal size/mm³ | 0.035 × 0.052 × 0.127 |
| Radiation | CuK*α* ( *λ* = 1.54184) |
| Independent reflections | 26117 [Rᵢₙₜ = 0.1666, R_{sigma} = 0.0978] |
| Goodness-of-fit on F² | 1.077 |
| Final R indexes [I>=2σ (I)] | R₁ = 0.1130, wR₂ = 0.3013 |

As shown in Fig. 9, PCNII-1 has a unit structure formed from a five-nuclear cluster including six TPHAP-derived pyridyl groups, eight MA-derived carboxyl groups, and five cobalt ions. The PCN according to Example 5 had isolated pores as shown in Fig. 10, with a maximum opening size of 12.42 × 12.40 Å.

### [Example 6]

7.3 mg (0.03 mmol) of 4,4'-biphenyldicarboxylic acid (H₂BPDC), 6.4 mg (0.01 mmol) of tetra-n-butylammonium 2,5,8-tri(4'-pyridyl)1,3,4,6,7,9-hexaazaphenalenyl (TBA⁺TPHAP⁻), and 6.6 mg (0.03 mmol) of cobalt bromide were heated with a mixed solvent of 2 cm³ of DMF and 0.1 cm³ of methanol (MeOH) in a vial at 80°C for 24 hours to provide square reddish purple PCN single crystals (PCNIII-1).

The resulting orange single crystal PCNIII-1 was identified by X-ray structural analysis and confirmed to be a PCN having a unit structure represented by [(Co²⁺)₅(TPHAP⁻)₄(BPDC²⁻)₄]·(TBA⁺)₂.

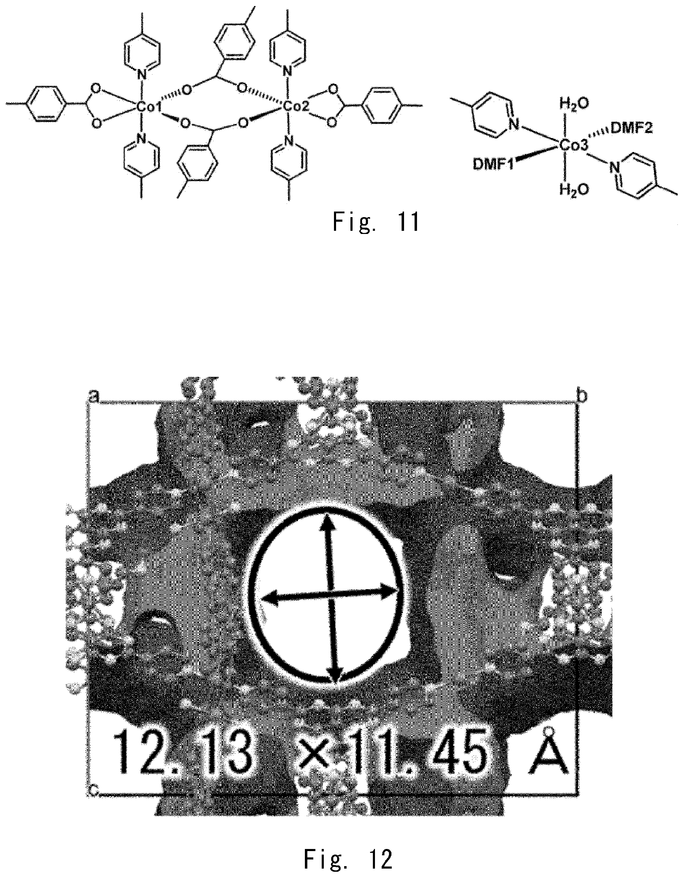

The results of X-ray structural analysis of PCNIII-1 are shown in Table 6, Fig. 11, and Fig. 12. The values in parentheses in Table 6 indicate statistical errors for the values in the table.

### [Table 6]

**Table 6**

| Name | PCNIII-1 |
|---|---|
| Temperature/K | 173.15 |
| Crystal system | Monoclinic |
| Space group | P2₁/n |
| a/Å | 19.9901 (12) |
| b/Å | 31.4742(16) |
| c/Å | 26.6899(18) |
| *α*/° | 90 |
| *β*/° | 108.513(7) |
| *γ*/° | 90 |
| Volume/Å³ | 15923.6(18) |
| Z | 4 |
| Crystal size/mm³ | 0.024 × 0.051 × 0.168 |
| Radiation | Mo K*α* ( *λ* = 0.71073) |
| Independent reflections | 36746 [Rᵢₙₜ = 0.4673, R_{sigma} = 0.4628] |
| Goodness-of-fit on F² | 1.139 |
| Final R indexes [I>=2σ (I)] | R₁ = 0.1973, wR₂ = 0.4539 |

As shown in Fig. 11, PCNIII-1 has a unit structure formed from a cobalt dinuclear cluster including four TPHAP-derived pyridyl groups and four BPDC-derived carboxy groups and from a cobalt mononuclear cluster including two TPHAP-derived pyridyl groups, two DMFs, and two waters. PCNIII-1 according to Example 6 had pores as shown in Fig. 12, with a maximum opening size of 12.13 × 11.45 Å.

### [Example 7]

5.9 mg (0.03 mmol) of 2,5-dihydroxyterephthalic acid (H₂DHC), 6.4 mg (0.01 mmol) of tetra-n-butylammonium 2,5,8-tri(4'-pyridyl)1,3,4,6,7,9-hexaazaphenalenyl (TBA⁺TPHAP⁻), and 6.6 mg (0.03 mmol) of cobalt bromide were heated with 2 cm³ of DMF in a vial at 80°C for 18 hours to provide square orange PCN single crystals (PCNIV-1).

The resulting orange single crystal PCNIV-1 was identified by X-ray structural analysis and confirmed to be a PCN having a composition represented by [(Co²⁺)₂(TPHAP⁻)₁(DHC²⁻)₂]·TBA⁺.

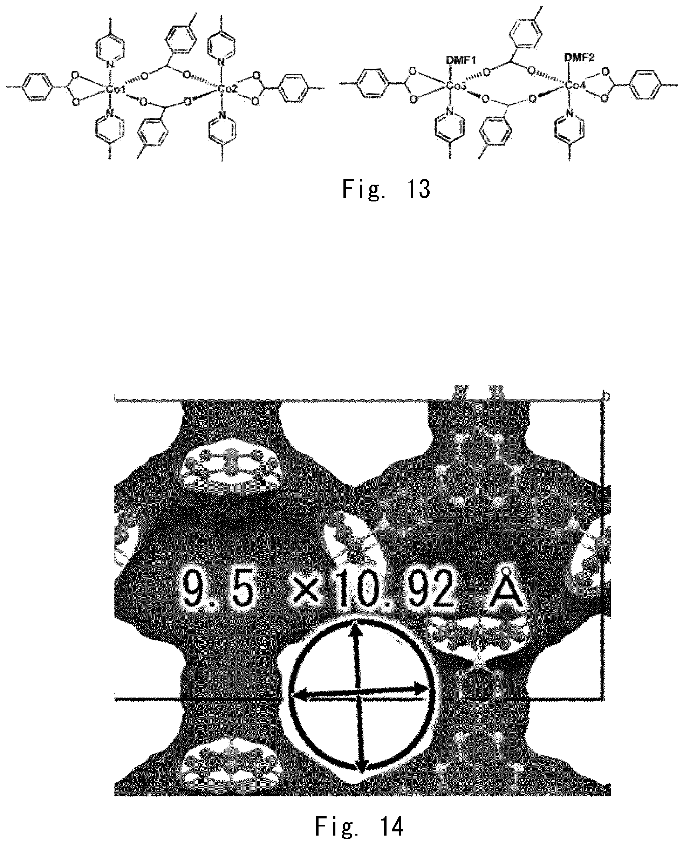

The results of the X-ray structural analysis of PCNIV-1 are shown in Table 7, Fig. 13 and Fig 14.

### [Table 7]

**Table 7**

| Name | PCNIV-1 |
|---|---|
| Temperature/K | 173.15 |
| Crystal system | monoclinic |
| Space group | P2₁/m |
| a/Å | 14.7620(14) |
| b/Å | 30.938 (2) |
| c/Å | 18.7627(16) |
| *α*/° | 90 |
| *β*/° | 92.924(9) |
| *γ*/° | 90 |
| Volume/Å³ | 8558.0(13) |
| Z | 4 |
| Crystal size/mm³ | 0.260 × 0.251 × 0.037 |
| Radiation | Mo K*α* (*λ* = 0.71073) |
| Independent reflections | 20131 [Rᵢₙₜ = 0.2971, R_{sigma} = 0.2442] |
| Goodness-of-fit on F² | 1.773 |
| Final R indexes [I>=2σ (I)] | R₁ = 0.3044, wR₂ = 0.6383 |

PCNIV-1 includes two types of cobalt dinuclear clusters as shown in Fig. 13, and one is a cobalt dinuclear cluster including four TPHAP-derived pyridyl groups and four DHC-derived carboxy groups, and the other is a cobalt dinuclear cluster including two TPHAP-derived pyridyl groups, two DMFs, and four DHC-derived carboxy groups. The PCN of Example 7 had pores as shown in Fig. 14, with a maximum opening size of 10.92 × 9.50 Å.

### [Example 8]

71.8 mg (0.1 mmol) of 5',5"-bis(4-carboxyphenyl)-2',2",4',4",6',6"-hexamethylene-[1,1':3',1":3",1‴-quaterphenyl]-4,4‴-dicarboxylic acid (H₄TCBM), 64 mg (0.1 mmol) of tetra-n-butylammonium 2,5,8-tri(4'-pyridyl)1,3,4,6,7,9-hexaazaphenalenyl (TBA⁺TPHAP⁻), and 43.7 mg (0.2 mmol) of cobalt bromide were heated with 20 cm³ of dehydrated DMF at 100°C in a pressure vessel for 72 hours to provide reddish purple single crystals of PCNV-1.

The resulting PCNV-1 was identified by single crystal X-ray diffraction and confirmed to be a PCN having a unit structure represented by (Co²⁺)₂(TPHAP⁻)(TCBM³⁻).

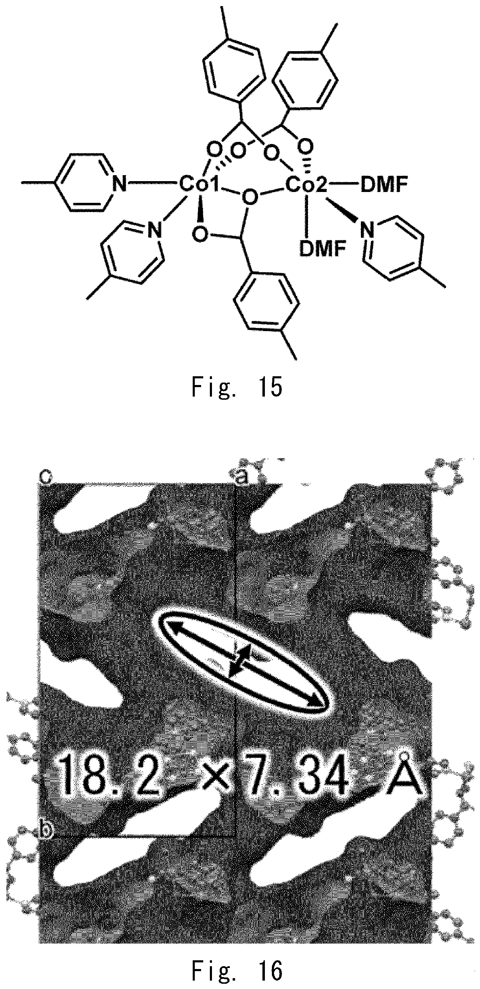

The results of X-ray structural analysis of PCNV-1 are shown in Table 8, Fig. 15, and Fig. 16.

### [Table 8]

**Table 8**

| Name | PCNV-1 |
|---|---|
| Temperature/K | 95 |
| Crystal system | Monoclinic |
| Space group | P2₁/n |
| a/Å | 18.203(4) |
| b/Å | 32.801(6) |
| c/Å | 23.671(2) |
| *α*/° | 90 |
| *β*/° | 93.188 (9) |
| *γ*/° | 90 |
| Volume/Å³ | 14112(4) |
| Z | 4 |
| Crystal size/mm³ | 0.477 × 0.181 × 0.027 |
| Radiation | 0.75Å (KEK BL-5A) |
| Independent reflections | 48027 [Rᵢₙₜ = 0.0353, R_{sigma} = 0.0224] |
| Goodness-of-fit on F² | 1.925 |
| Final R indexes [I>=2σ (I)] | R₁ = 0.1552, wR₂ = 0.4646 |

It was confirmed that PCNV-1 has a unit structure formed from a cobalt dinuclear cluster including four TPHAP-derived pyridine sites, four TCBM-derived carboxyl groups, and two DMFs, as shown in Fig. 15. The PCN of Example 8 has pores as shown in Fig. 16, with a maximum opening size of 7.34 × 18.20 Å.

### [Example 9]

8.6 mg (0.03 mmol) of [1,1'-biphenyl]-3,4',5-tricarboxylic acid (H₃BTCA), 6.6 mg (0.03 mmol) of tetra-n-butylammonium 2,5,8-tri(4'-pyridyl)1,3,4,6,7,9-hexaazaphenalenyl (TBA⁺TPHAP⁻), and 8.6 mg (0.03 mmol) of cobalt bromide were heated with 2 cm³ of dehydrated DMF in a vial at 100°C for 72 hours to provide reddish purple single crystals of PCNVI-1.

The resulting PCNVI-1 was identified by single crystal X-ray diffraction and confirmed to be a PCN having a unit structure represented by [(Co²⁺)₃(TPHAP⁻)_{(4+2x)/3}(BTCA³⁻ )₂]·Y_{(4+2x)/3} (where m is any integer). However, 0 < x ≤ 1. It was confirmed that the unit structure is [(Co²⁺)₃(TPHAP⁻)₂(BTCA³⁻)₂]·TBA⁺₂.

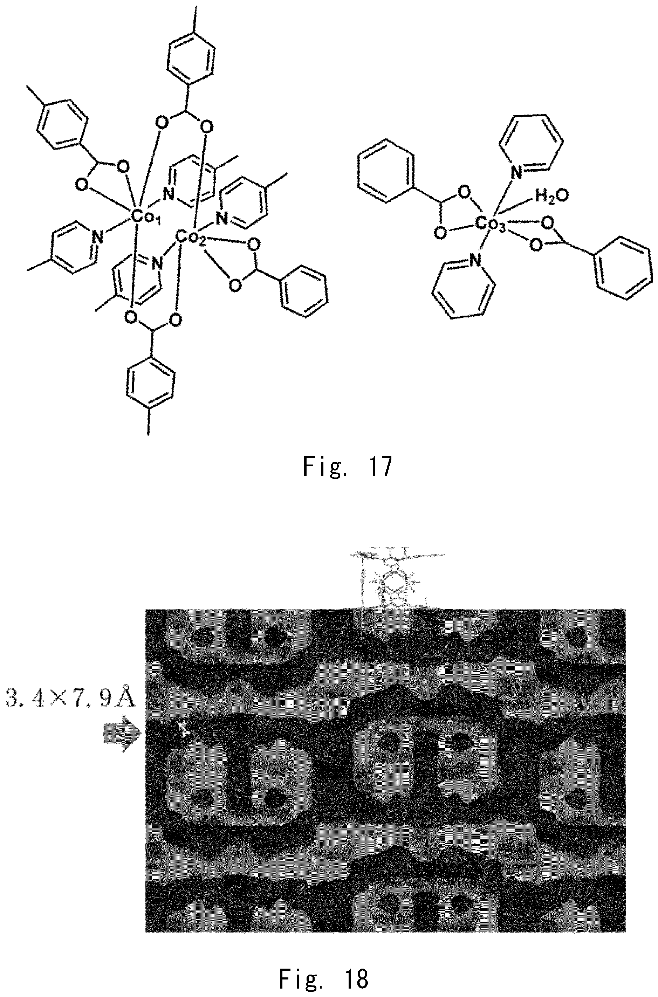

The results of the X-ray structural analysis of PCNVI-1 are shown in Table 9, Fig. 17, and Fig. 18.

### [Table 9]

**Table 9**

| NAME | PCNVI-1 |
|---|---|
| Temperature/K | 123.15 |
| Crystal system | Trigonal |
| Space group | R-3c |
| a/Å | 31.1843(6) |
| b/Å | 31.1843(6) |
| c/Å | 62.2037(18) |
| *α*/° | 90 |
| *β*/° | 90 |
| *γ*/° | 120 |
| Volume/Å3 | 52386(3) |
| Z | 36 |
| Radiation | MoK*_{α}* ( *λ* = 0.71073) |
| Independent reflections | 16048 [Rᵢₙₜ = 0.0579, R_{sigma} = 0.0482] |
| Goodness-of-fit on F2 | 1.569 |
| Final R indexes [I>=2σ (I)] | R1 = 0.1652, wR2 = 0.4540 |

PCNVI-1 has a unit structure, the structure formed by a cobalt dinuclear cluster formed from four TPHAP-derived pyridine sites and four BTCA-derived carboxy groups, as well as a cobalt mononuclear cluster formed from two TPHAP-derived pyridyl groups, two BTCA-derived carboxy groups, and one molecule of water, as shown in Fig. 17. The PCN of Example 9 has pores as shown in Fig. 18, with a maximum opening size of 3.40 × 7.90 Å

### [Example 10]

8.9 mg (16.7 mol) of 2,5,8-tri(4'-carboxybenzyl)-1,3,4,6,7,9-hexaazaphenalene (H₄TCHAP), 4.3 mg (24.4 mol) of 2,5-thiophenedicarboxylic acid (H₂TDC), 3.6 mg (52.1 mol) of 1H-1,2,3-triazole (HTAZ), and 26.6 mg (83.33 mol) of zinc iodide (II) were placed in a pressure vessel, 2 cm³ of dehydrated DMF and 0.030 cm³ of acetic acid were added thereto, and the mixture was heated at 120°C for 40 hours to provide colorless crystals of PCNVII-1.

The resulting PCNVII-1 was identified by single crystal X-ray diffraction and confirmed to be a PCN with a unit structure represented by [(Zn²⁺)₈(HTCHAP³⁻)₂(TDC²⁻)₃(TAZ⁻)₆]·(Y⁺)₂. Immediately after synthesis, the cation Y⁺ is a dimethylammonium ion (DMA⁺) generated by decomposition of the solvent N,N-dimethylformamide.

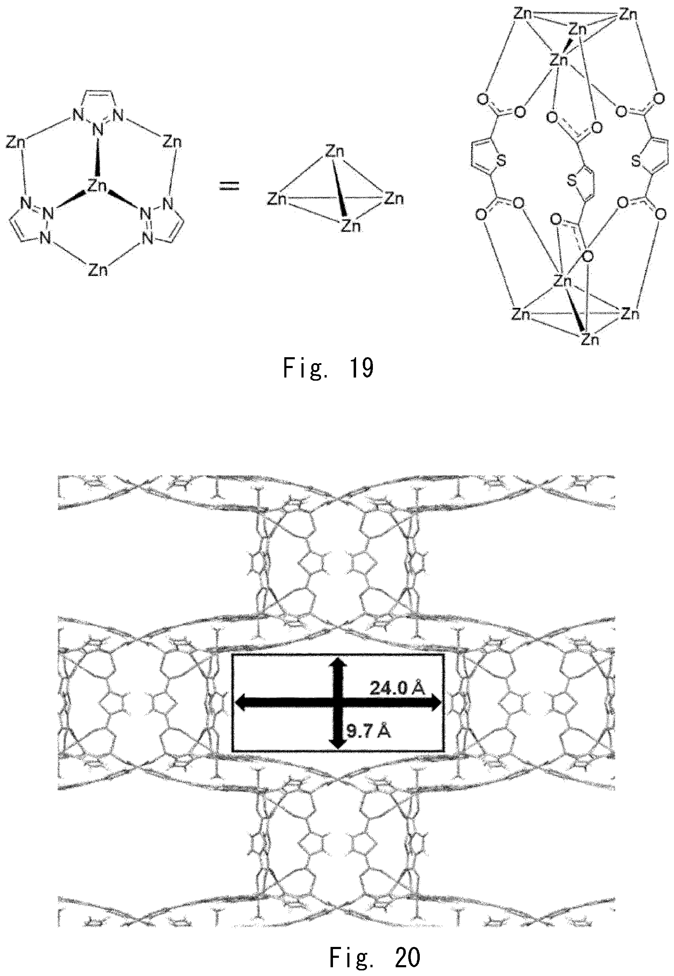

The results of the X-ray structural analysis of PCNVII-1 are shown in Table 10, Fig. 19, and Fig. 20.

### [Table 10]

**Table 10**

| Empirical formula | PCNVII-1 |
|---|---|
| Temperature/K | 123.15 |
| Crystal system | monoclinic |
| Space group | C2/m |
| a/Å | 47.9530(4) |
| b/Å | 30.0035(6) |
| c/Å | 24.7272(2) |
| *α*/° | 90 |
| *β*/° | 120.5970(10) |
| *γ*/° | 90 |
| Volume/Å³ | 30623.1(8) |
| Z | 8 |
| Crystal size/mm³ | 0.131 × 0.131 × 0.02 |
| Radiation | CuK*α* ( *λ* = 1. 54178) |
| Independent reflections | 32034 [Rᵢₙₜ = 0.0548, R_{sigma} = 0.0395] |
| Goodness-of-fit on F² | 1.315 |
| Final R indexes [I>=2σ (I)] | R₁ = 0.1060, wR₂ = 0.3266 |

The PCN of Example 10 had pores as shown in Fig. 20, with a maximum opening size of 9.70 × 24.00 Å. As shown in Fig. 19, PCNVII-1 had a structure in which metal clusters including eight zinc ions, three molecules of 2,5-thiophenedicarboxylic acid, and six molecules of 1,2,3-triazole were formed, and these were cross-linked by TCHAP.

The PCNs synthesized in Examples 1 to 10 were stable in DMF for more than one week. Others that showed notable stability are shown below. When the solvent stability of PCNI-3 in Example 3 was evaluated, it was found to be stable in acetonitrile, ethyl acetate, acetone, heptane, kerosene, octane, cyclooctane, cyclohexane, toluene, monochlorobenzene, mesitylene, 2,2,4-trimethylpentane, and tert-butyl methyl ether. Among these solvents, it was stable for about five days in ethyl acetate, and for more than one month in other organic solvents. When the solvent stability of PCNV-1 in Example 8 was evaluated, PCNV-1 was stable in methanol, ethyl acetate, acetone, hexane, heptane, kerosene, and cyclohexane for at least 5 days or more. In particular, it was confirmed that single crystals remained in solvents other than methanol after one month or more. When the solvent stability of PCNVII-1 in Example 10 was evaluated, it was confirmed that PCNVII-1 was stable in acetone and heptane and maintained single crystallinity in these solvents for one week or more.

An example of guest inclusion structure analysis using the synthesized PCN is described below.

### [Example 11]

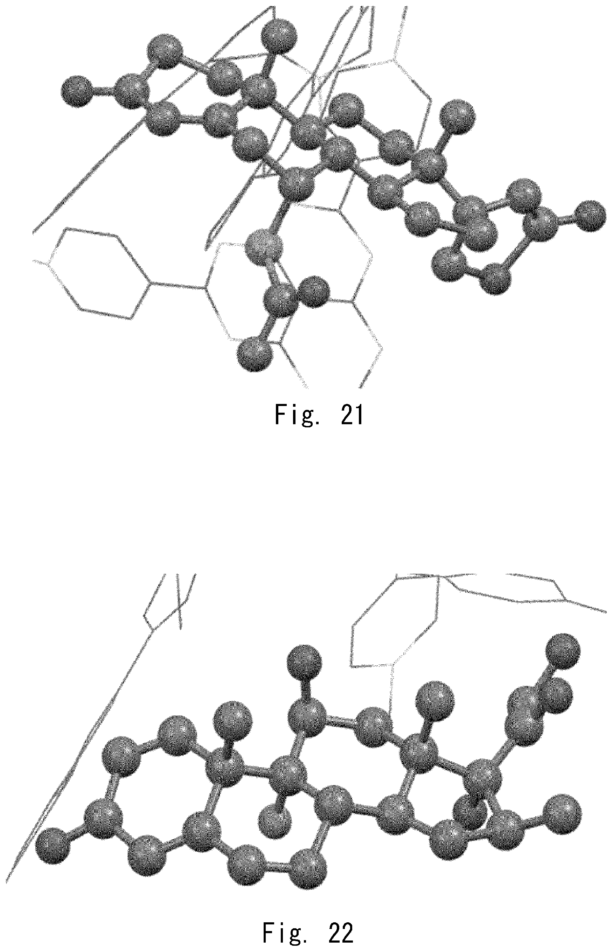

The PCNI-3 obtained in Example 3 was used to analyze the crystal structure of the guest molecule spironolactone (C₂₄H₃₂O₄S, 416.57 g/mol). First, PCNI-3 was washed with ethyl acetate three times and solvent-exchanged for one day. Then, immediately before the inclusion of spironolactone, ethyl acetate was replaced with heptane as the solvent. 2 mg of spironolactone was dissolved in a mixed solvent of 1 cm³ of heptane and 0.1 cm³ of acetone, several PCNI-3 crystals were added to this mixed solvent, the mixture was left at 40°C for 3 days and then spironolactone was included in PCNI-3, and single crystal X-ray diffraction was measured. The measurement results are shown in Table 11 and Fig. 21.

### [Table 11]

**Table 11**

| Name | inclusion of Spironolactone PCNI-3 |
|---|---|
| Temperature/K | 95 |
| Crystal system | monoclinic |
| Space group | P2₁ |
| a/Å | 32.060 (4) |
| b/Å | 19.739 (4) |
| c/Å | 35.105 (5) |
| α/° | 90 |
| *β*/° | 96.336 (2) |
| *γ*/° | 90 |
| Volume/Å³ | 22080 (6) |
| Z | 2 |
| Crystal size/mm³ | 0.146 × 0.139 × 0.021 |
| Radiation | 0.75Å (KEK BL-5A) |
| Independent reflections | 163418 [Rᵢₙₜ = 0.0772, R_{sigma} = 0.0862] |
| Data/restraints/parameters | 163418/3/2765 |
| Goodness-of-fit on F² | 1.532 |
| Final R indexes [I>=2σ (I)] | R₁ = 0.1680, wR₂ = 0.4379 |
| Flack parameter | 0.107(4) |

### [Example 12]

The PCNI-3 obtained in Example 3 was used to analyze the crystal structure of the guest molecule betamethasone (C₂₂H₂₉FO₅, 392.46 g/mol). First, PCNI-3 was washed three times with ethyl acetate and solvent-exchanged for one day. Then, immediately before the inclusion of betamethasone, ethyl acetate was exchanged for toluene as the solvent. 2 mg of betamethasone was dissolved in 1 cm³ of toluene, several PCNI-3 crystals were added to this solution, the mixture was left to stand at 40°C for three days and then betamethasone was included in PCNI-3, and single crystal X-ray diffraction was measured. The measurement results are shown in Table 12 and Fig. 22.

### [Table 12]

**Table 12**

| Name | inclusion of betamethasone PCNI-3 |
|---|---|
| Temperature/K | 95 |
| Crystal system | orthorhombic |
| Space group | P2₁2₁2₁ |
| a/Å | 16.361(4) |
| b/Å | 34.981(10) |
| c/Å | 38.823(15) |
| α/° | 90 |
| *β*/° | 90 |
| *γ*/° | 90 |
| Volume/Å³ | 22219(12) |
| Z | 4 |
| Crystal size/mm³ | 0.098 × 0.186 × 0.031 |
| Radiation | 0.75Å (KEK BL-5A) |
| 2*θ* range for data collection/° | 1.654 to 71.382 |
| Index ranges | -25 ≤ h ≤ 25, -52 ≤ k ≤ 52, -59 ≤ 1 ≤ 60 |
| Reflections collected | 489415 |
| Independent reflections | 84720 [Rᵢₙₜ = 0.0960, R_{sigma} = 0.0677] |
| Data/restraints/parameters | 84720/877/2035 |
| Goodness-of-fit on F² | 1.052 |
| Final R indexes [I>=2σ (I)] | R₁ = 0.1232, wR₂ = 0.3128 |
| Final R indexes [all data] | R₁ = 0.2304, wR₂ = 0.3868 |
| Largest diff. peak/hole / e Å⁻³ | 1.34/-0.98 |
| Flack parameter | 0.023(5) |

### [Example 13]

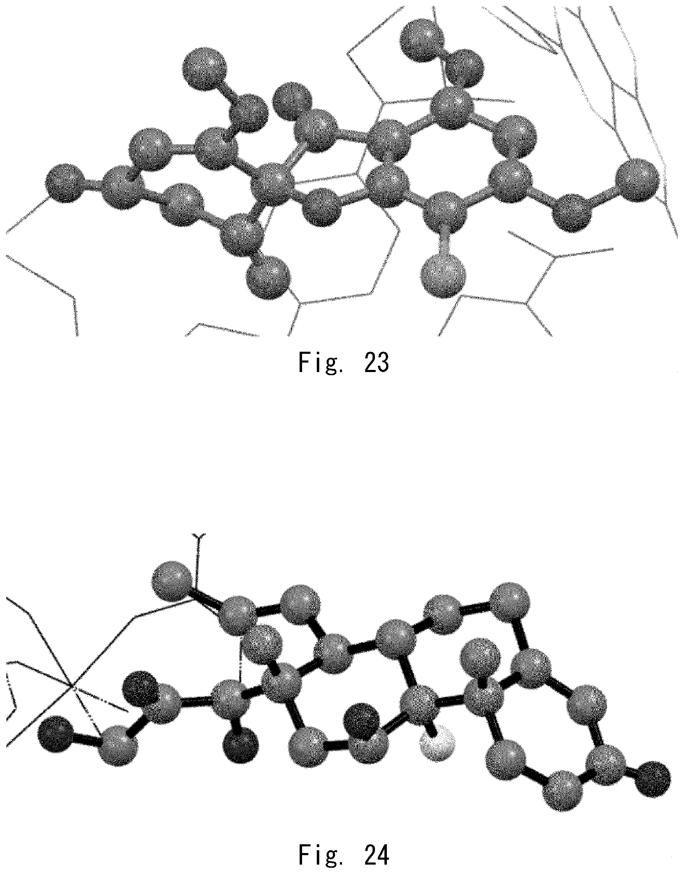

The PCNV-1 obtained in Example 8 was used to analyze the crystal structure of the guest molecule griseofulvin (C₁₇H₁₇ClO₆, 352.77 g/mol). First, PCNV-1 was washed three times with ethyl acetate and solvent-exchanged for one day. Then, immediately before the inclusion of griseofulvin, ethyl acetate was replaced with hexane as the solvent. 2 mg of griseofulvin was added to 1 cm³ of n-heptane, several PCNV-1 crystals were added to this solution, the mixture was allowed to stand at 40°C for three days and then griseofulvin was included in PCNV-1, and single crystal X-ray diffraction was measured. The measurement results are shown in Table 13 and Fig. 23.

### [Table 13]

**Table 13**

| Empirical formula | inclusion of griseofulvin PCNV-1 |
|---|---|
| Temperature/K | 95 |
| Crystal system | monoclinic |
| Space group | P2₁ |
| a/Å | 18.298(2) |
| b/Å | 34.540 (10) |
| c/Å | 24.327(5) |
| *α*/° | 90 |
| *β*/° | 95.913(6) |
| *γ*/° | 90 |
| Volume/Å³ | 15293 (5) |
| Z | 2 |
| Crystal size/mm³ | 0.267 × 0.271 × 0.053 |
| Radiation | 0.75Å (KEK BL-5A) |
| Independent reflections | 104174 [Rᵢₙₜ = 0.0332, R_{sigma} = 0.0341] |
| Goodness-of-fit on F² | 1.569 |
| Final R indexes [I>=2σ (I)] | R₁ = 0.1424, wR₂ = 0.4122 |
| Flack parameter | 0.320 (4) |

### [Example 14]

The PCNVII-1 obtained in Example 10 was used to analyze the crystal structure of the guest molecule betamethasone (C₂₂H₂₉FO₅, 392.46 g/mol). First, PCNVII-1 was washed three times with DMF, then the solvent was replaced with acetone and left at room temperature for three days. Then, several PCNVII-1 crystals were added to a solution in which betamethasone was dissolved to saturation amount in 1 cm³ of heptane, and betamethasone was included. After three days at 40°C, structure analysis was performed by single crystal X-ray structure analysis (KEK BL-5A). The measurement results are shown in Table 14 and Fig. 24.

### [Table 14]

**Table 14**

| Name | inclusion of betamethasone PCNVII-1 |
|---|---|
| Temperature/K | 95 |
| Crystal system | Monoclinic |
| Space group | C2 |
| a/Å | 46.524(11) |
| b/Å | 31.572(5) |
| c/Å | 24.832(7) |
| *α*/° | 90 |
| *β*/° | 119.635(14) |
| *γ*/° | 90 |
| Volume/Å³ | 31703(14) |
| Z | 4 |
| Radiation | 0.75Å (KEK BL-5A) |
| Independent reflections | 103642 [Rᵢₙₜ = 0.0840, R_{sigma} = 0.0776] |
| Goodness-of-fit on F² | 0.945 |
| Final R indexes [I>=2σ (I)] | R₁ = 0.1174, wR₂ = 0.2977 |
| Flack parameter | 0.204 (8) |

### [Example 15]

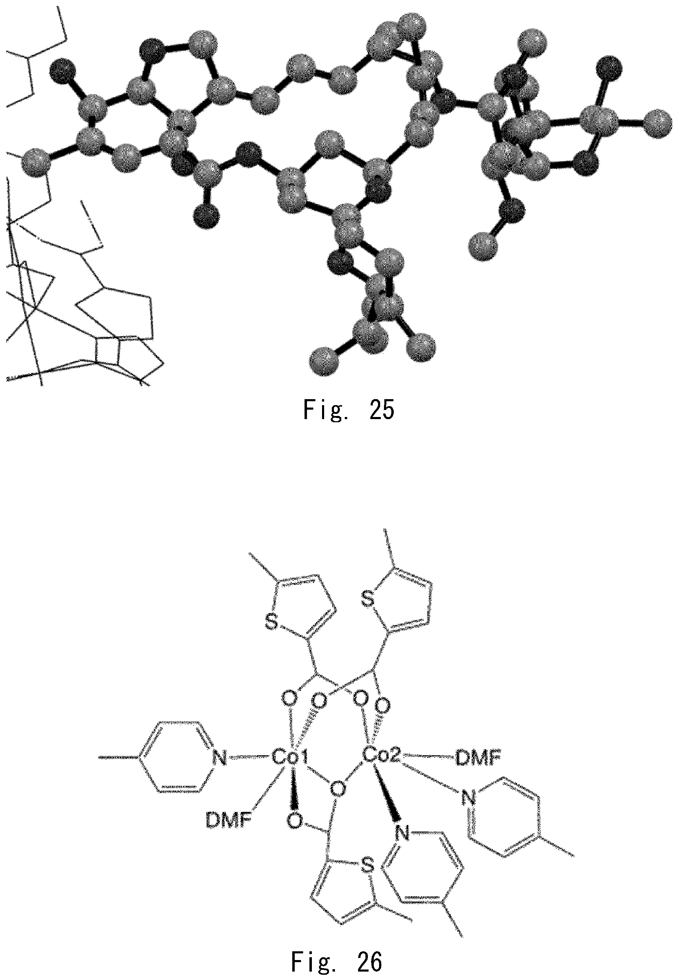

The PCNVII-1 obtained in Example 10 was used to analyze the crystal structure of the guest molecule ivermectin (C₄₈H₇₄O₁₄, 875.11 g/mol). First, PCNVII-1 was washed three times with DMF, then the solvent was replaced with acetone and left at room temperature for three days. Then, several PCNVII-1 crystals were added to a solution in which ivermectin had been dissolved to a saturation amount in a mixture of 0.1 cm³ of acetone and 1 cm³ of heptane, and ivermectin was included. After three days at room temperature, single crystal X-ray structure analysis was performed. The measurement results are shown in Table 15 and Fig. 25.

### [Table 15]

**Table 15**

| Name | inclusion of ivermectin PCNVII-1 |
|---|---|
| Temperature/K | 95 |
| Crystal system | Monoclinic |
| Space group | C2 |
| a/Å | 47.340(9) |
| b/Å | 30.700(3) |
| c/Å | 24.975(4) |
| *α*/° | 90 |
| *β*/° | 121.568(4) |
| *γ*/° | 90 |
| Volume/Å³ | 30926(8) |
| Z | 4 |
| Crystal size/mm³ | 0.18 × 0.08 × 0.02 |
| Radiation | 0.75Å (KEK BL-5A) |
| Independent reflections | 102425 [Rᵢₙₜ = 0.0630, R_{sigma} = 0.0573] |
| Goodness-of-fit on F² | 1.107 |
| Final R indexes [I>=2σ (I)] | R₁ = 0.1207, wR₂ = 0.3281 |
| Flack parameter | 0.096 (5) |

As shown in Fig. 21 to Fig. 25 and the like, it was confirmed that the guest molecules, which are the compounds to be analyzed, were included within various PCNs, and the structures thereof could be analyzed.

### [Example 16]

5.2 mg (0.03 mmol) of 2,5-thiophenedicarboxylic acid (H₂TDCA), 6.4 mg (0.01 mmol) of 2,5,8-tri(4'-pyridyl)1,3,4,6,7,9-hexaazaphenalenyl (4TPHAP⁻), and 6.6 mg (0.03 mmol) of cobalt bromide were heated in a mixed solution of 2 mL of DMF and 100 µL of acetic acid at 100°C in a vial for 72 hours to provide square reddish purple PCN single crystals (PCNI-5, TDCA-PCN).

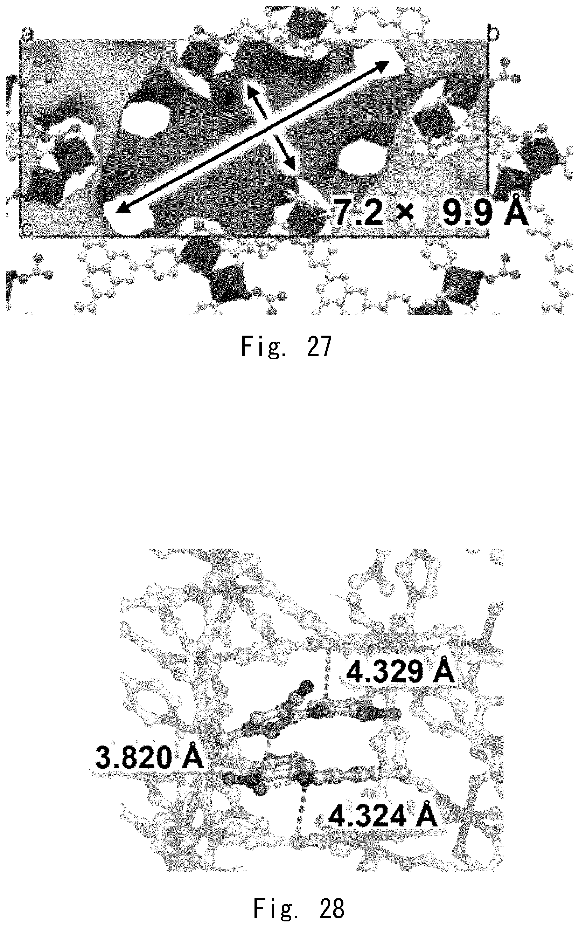

The resulting reddish purple PCN single crystal, PCNI-5, was identified by X-ray structure analysis and confirmed to be a PCN having a unit structure represented by (Co²⁺)₄(TPHAP⁻)₂(TDCA²⁻)₃. The results of the X-ray structure analysis of PCNI-5 are shown in Table 16, Fig. 26, and Fig. 27.

### [Table 16]

**Table 16**

| Name | PCNI-5 |
|---|---|
| Temperature/K | 173.15 |
| Crystal system | Monoclinic |
| Space group | P2₁/n |
| a/Å | 9.6843(3) |
| b/Å | 41.3785(12) |
| c/Å | 18.0270(5) |
| *α*/° | 90 |
| *β*/° | 104.853(3) |
| *γ*/° | 90 |
| Volume/Å³ | 6982.4(4) |
| Z | 1 |
| Crystal size/mm³ | 0.075 × 0.045 × 0.0225 |
| Radiation | Mo K*α* (*λ* = 0.71073) |
| Independent reflections | 14138 [Rint = 0.0815, Rsigma = 0.0608] |
| Goodness-of-fit on F² | 1.295 |
| Final R indexes [I>=2*σ* (I)] | R1 = 0.1270, wR2 = 0.3409 |

PCNI-5 is formed based on a cobalt dinuclear cluster including three TPHAP-derived pyridyl groups, three BDC-derived carboxylic acids, and two DMFs, as shown in Fig. 26. The PCN of Example 16 has pores as shown in Fig. 27, with a maximum opening size of 7.2 × 9.9 Å. In addition, the porosity was 50.6% (3536 Å³).

### [Example 17]

The PCNI-5 obtained in Example 16 was used to analyze the crystal structure of the guest molecule 5-methyl-2-[(2-nitrophenyl)amino]-3-thiophenecarbonitrile (ROY). First, PCNI-5 was washed three times with ethyl acetate and solvent-exchanged for one day. Then, immediately before the inclusion of ROY, ethyl acetate was replaced with heptane as the solvent. 1 mg of ROY was dissolved in 1 cm³ of heptane, several particles of PCNI-5 were added to this solution, the mixture was allowed to stand at 40°C for three days and then ROY was included in PCNI-5, and single crystal X-ray diffraction was measured. The measurement results are shown in Table 17 and Fig. 28.

### [Table 17]

**Table 17**

| Name | inclusion of ROY PCNI-5 |
|---|---|
| Temperature/K | 95 |
| Crystal system | Monoclinic |
| Space group | P2₁/n |
| a/Å | 19.113(4) |
| b/Å | 41.470(3) |
| c/Å | 19.362(3) |
| *α*/° | 90 |
| *β*/° | 111.355(10) |
| *γ*/° | 90 |
| Volume/Å³ | 14293(4) |
| Z | 1 |
| Crystal size/mm³ | 0.075 × 0.045 × 0.0225 |
| Radiation | 0.75Å (KEK BL-5A) |
| 2*θ* range for data collection/° | 2.072 to 71.166 |
| Index ranges | -25 ≤ h ≤ 26, -61 ≤ k ≤ 60, -29 ≤ 1 ≤ 29 |
| Reflections collected | 157495 |
| Independent reflections | 46366 [Rint = 0.0545, Rsigma = 0.0491] |
| Data/restraints/parameters | 46366/0/1853 |
| Goodness-of-fit on F² | 1.265 |
| Final R indexes [I>=2*σ* (I)] | R1 = 0.1034, wR2 = 0.3152 |
| Final R indexes [all data] | R1 = 0.1408, wR2 = 0.3476 |
| Largest diff. peak/hole / e Å⁻³ | 2.57/-3.48 |

### [Example 18]

4.7 mg (0.03 mmol) of 2,5-furandicarboxylic acid (H₂FDCA), 6.4 mg (0.01 mmol) of 2,5,8-tri(4'-pyridyl)1,3,4,6,7,9-hexaazaphenalenyl (4TPHAP-), and 6.6 mg (0.03 mmol) of cobalt bromide were heated in a mixed solution of 2 mL of DMF and 100 µL of acetic acid in a vial at 100°C for 72 hours to provide square reddish purple PCN single crystals (PCNVIII-1, FDCA-PCN).

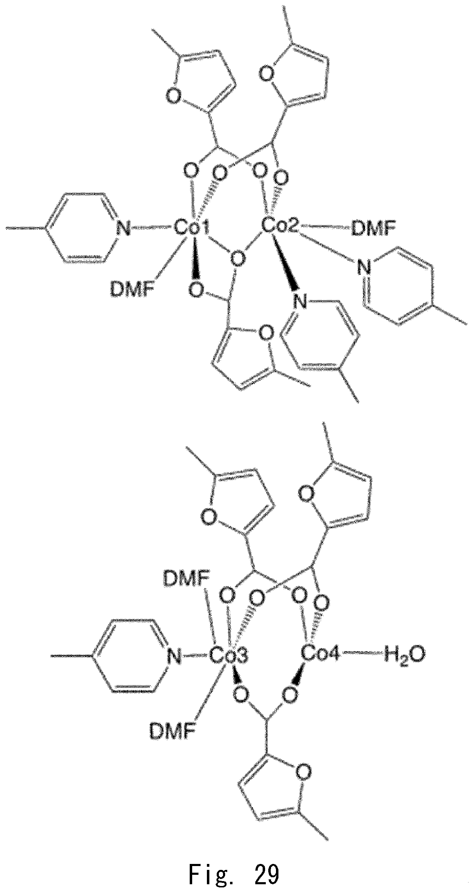

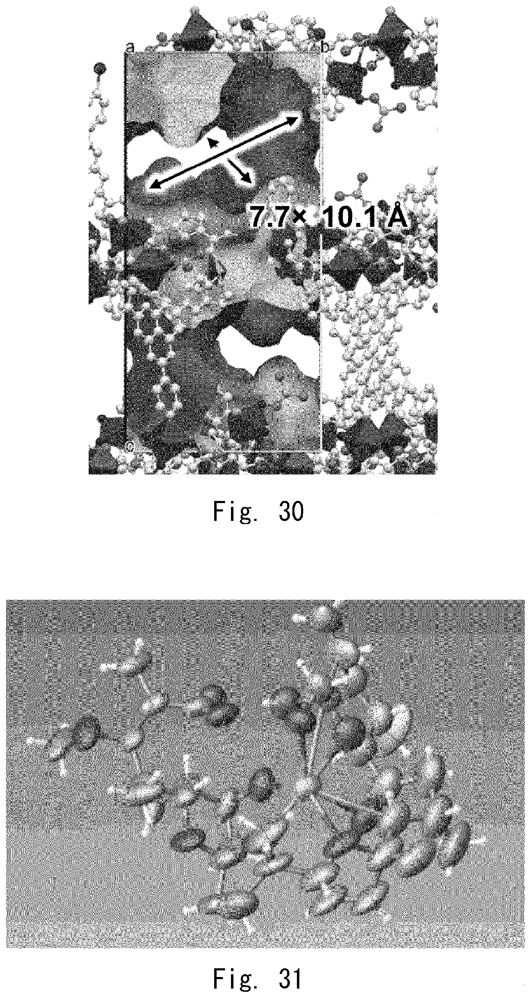

The resulting reddish purple PCN single crystal, PCNVIII-1, was identified by X-ray structure analysis and confirmed to be a PCN having a unit structure represented by (Co²⁺)₅(Co³⁺)₁(TPHAP⁻)₃(FDCA²⁻)₅. The results of the X-ray structure analysis of PCN-18 are shown in Table 18, Fig. 29, and Fig. 30.

### [Table 18]

**Table 18**

| Name | PCNVIII-1 |
|---|---|
| Temperature/K | 173.15 |
| Crystal system | Monoclinic |
| Space group | Cm |
| a/Å | 21.0227(9) |
| b/Å | 61.8945(14) |
| c/Å | 9.2701(4) |
| *α*/° | 90 |
| *β*/° | 120.985(6) |
| *γ*/° | 90 |
| Volume/Å³ | 10340.9(9) |
| Z | 1 |
| Crystal size/mm³ | 0.199 × 0.209 × 0.0596 |
| Radiation | Mo K*α* ( *λ* = 0.71073) |
| Independent reflections | 17529 [Rint = 0.0503, Rsigma = 0.0738] |
| Goodness-of-fit on F² | 1.010 |
| Final R indexes [I>=2 *σ* (I)] | R1 = 0.0669, wR2 = 0.1899 |

PCNVIII-1 according to Example 18 had pores as shown in Fig. 30, with a maximum opening size of 7.7 × 10.1 Å. The porosity was 49.7% (6092.8 Å³).

### [Example 19]

The PCNV-1 obtained in Example 8 was used to analyze the crystal structure of the guest molecule monensin sodium (C₃₆H₆₁NaO₁₁, 682.86 g/mol). First, PCNV-1 was washed three times with ethyl acetate and solvent-exchanged for one day. Then, immediately before the inclusion of monensin sodium, ethyl acetate was replaced with heptane as the solvent. 1 mg of monensin sodium was added to 1 cm³ of heptane, several PCNV-1 crystals were added to this solution, the mixture was left to stand at 40°C for three days and then monensin sodium was included in PCNV-1, and single crystal X-ray diffraction was measured.

The measurement results are shown in Table 19 and Fig. 31.

### [Table 19]

**Table 19**

| Empirical formula | inclusion of monensin sodium PCNV-1 |
|---|---|
| Temperature/K | 95 |
| Crystal system | monoclinic |
| Space group | P2₁ |
| a/Å | 18.222(2) |
| b/Å | 32.244(6) |
| c/Å | 23.684(3) |
| *α*/° | 90 |
| *β*/° | 92.966(13) |
| *γ*/° | 90 |
| Volume/Å³ | 15457(4) |
| Z | 2 |
| Crystal size/mm³ | 0.133 × 0.175 × 0.057 |
| Radiation | 0.75Å (KEK BL-5A) |
| Independent reflections | 101660 [Rᵢₙₜ = 0.0388, R_{sigma} = 0.0273] |
| Goodness-of-fit on F² | 1.144 |
| Final R indexes [I>=2 σ (I)] | R₁ = 0.11.39, wR₂ = 0.3435 |
| Flack parameter | 0.054(4) |

### Industrial Applicability

The present PCN can be used as a crystal for analyzing the crystal structure of high molecular weight guest molecules such as pharmaceuticals and peptides. In particular, it is expected that the structure of medium-sized molecules, which have been difficult to analyze until now, can be easily analyzed. Further, the present PCN can be used in substance purification processes, as a reaction field in closed spaces, as an adsorbent, and as a chemical sensor material.

This application claims priority based on Japanese Patent Application No. 2022-140327, filed on September 2, 2022, the disclosure of which is incorporated herein in its entirety.

## Claims

1. A porous coordination network which has a three-dimensional network structure and in which pores capable of encapsulating guest molecules are formed,
the porous coordination network comprising a crystal structure having a unit structure represented by any one of following formulas (I) to (VIII):
(M²⁺)₄(L1a⁻)₂(L2a²⁻)₃ formula (I)
(M²⁺)₁₁(L1a⁻)₆(L2a²⁻)₈ formula (II)
[(M²⁺)₅(L1a⁻)₄(L2a²⁻)₄]·Y₂ₚ formula (III)
[(M²⁺)₂(L1a)₁(L2a²⁻)₂]·Yₚ formula (IV)
(M²⁺)₂(L1a⁻)₁(L2b³⁻)₁ formula (V)
[(M²⁺)₃(L1a⁻)₂(L2b³⁻)₂]·Y₂ₚ formula (VI)
[(M²⁺)₈(L1b³⁻)₂(L2a²⁻)₃(L3⁻)₆]·Y₂ₚ formula (VII)
(M²⁺)₅(M³⁺)₁(L1a⁻)₃(L2a²⁻)₅ formula (VIII)
where M²⁺ represents a divalent metal ion, M³⁺ represents a trivalent metal ion,
L1a and L1b represent tridentate ligands having hexaazaphenalenyl,
L1a⁻ represents a first ligand ion of a following formula (1), or a first ligand ion in which 1 to 12 hydrogen atoms of an aromatic ring of the formula (1) are each independently replaced by a group selected from an alkyl group having 1 to 4 carbon atoms, a halogen, a hydroxyl group, and an amino group, 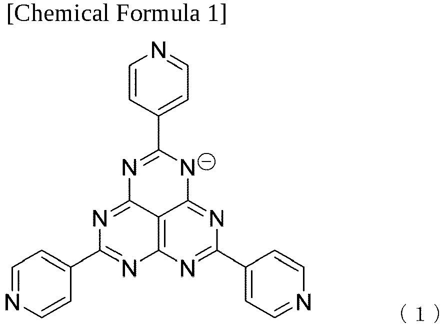
L1b³⁻ represents a first ligand ion represented by a following formula (2), or a first ligand ion in which 1 to 12 hydrogen atoms of an aromatic ring of the formula (2) are each independently replaced by a group selected from an alkyl group having 1 to 4 carbon atoms, a halogen, a hydroxyl group, and an amino group, 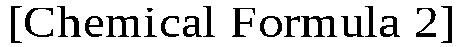 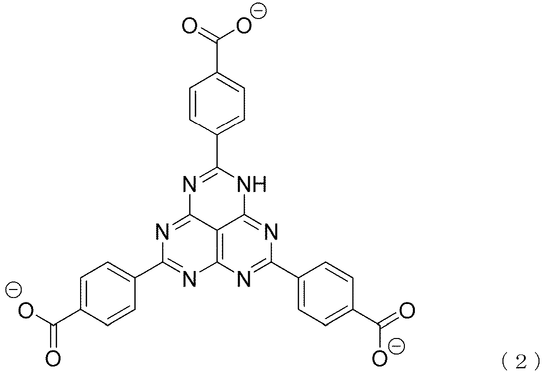
L2a represents a bidentate ligand containing two or more carboxy groups,
L2a²⁻ represents a second ligand ion in which two of the carboxy groups are coordinated to the metal ion as carboxylate anions,
L2b represents a tridentate ligand containing three or more carboxy groups,
L2b³⁻ represents a second ligand ion in which three of the carboxy groups are coordinated to the metal ion as carboxylate anions,
L3⁻ represents a third ligand ion that is an anion of triazole or a triazole derivative,
Y represents a cation, p represents 1/(charge of Y), and
a solvent is optionally further coordinated to the unit structures of formulas (I) to (VII).

2. The porous coordination network according to claim 1, wherein the metal is at least one selected from Mg, Ca, Mn, Fe, Co, Ni, Cu, Zn, Rh, Ag, Cd, Ir, Pt, and Au.

3. The porous coordination network according to claim 1, wherein
the L2a and the L2b each have a molecular weight of 90 to 1000 and are at least one selected from a following formula (3):
[Chemical Formula 3]
**R¹-(COOH)ₙ** (3)
where n represents an integer of 2 or more for the L2a, and represents an integer of 3 or more for the L2b, and
R¹ has at least any one of structures of
a linear or branched aliphatic group having 2 to 20 carbon atoms,
an aromatic group having 6 to 72 carbon atoms, and
a heterocycle having 2 to 60 carbon atoms,
optionally having an unsaturated bond, optionally having a bond including a heteroatom, and optionally having a substituent.

4. The porous coordination network according to claim 1, wherein the porous coordination network is used for guest molecule analysis.

5. The porous coordination network according to claim 1, wherein the guest molecule is a medium-sized molecule having a molecular weight of 50 to 7000.

6. A method for preparing a sample for crystal structure analysis, comprising:
providing a sample in which a compound to be analyzed is dissolved in a solvent;
dispersing the porous coordination network according to any one of claims 1 to 5 in the sample; and
incorporating the compound to be analyzed into pores of the porous coordination network.

7. A method for determining a molecular structure of a compound to be analyzed, comprising performing crystal structure analysis using a sample for crystal structure analysis obtained by the method for preparing a sample for crystal structure analysis according to claim 6.
